(19) Europäisches Patentamt
European Patent Office
Office européen des brevets

(11) EP 4 635 963 A1

(12) EUROPEAN PATENT APPLICATION
published in accordance with Art. 153(4) EPC

(43) Date of publication:
22.10.2025 Bulletin 2025/43

(21) Application number: 23903290.7

(22) Date of filing: 29.11.2023

(51) International Patent Classification (IPC):
*C07F 11/00* *(2006.01)* *C07C 25/02* *(2006.01)*
*C07C 381/12* *(2006.01)*

(52) Cooperative Patent Classification (CPC):
C07C 25/02; C07C 381/12; C07F 11/00

(86) International application number:
PCT/JP2023/042798

(87) International publication number:
WO 2024/128000 (20.06.2024 Gazette 2024/25)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 13.12.2022 JP 2022198954

(71) Applicant: TOKYO OHKA KOGYO CO., LTD.
Kawasaki-shi, Kanagawa 211 0012 (JP)

(72) Inventors:
- INARI, Takatoshi
Kawasaki-shi, Kanagawa 211-0012 (JP)
- NODA, Kunihiro
Kawasaki-shi, Kanagawa 211-0012 (JP)
- KUBO, Keisuke
Kawasaki-shi, Kanagawa 211-0012 (JP)
- NISHIZAWA, Akito
Kawasaki-shi, Kanagawa 211-0012 (JP)
- KIMURA, Kenta
Kawasaki-shi, Kanagawa 211-0012 (JP)
- KINOSHITA, Yohei
Kawasaki-shi, Kanagawa 211-0012 (JP)
- WAKIYA, Kazumasa
Kawasaki-shi, Kanagawa 211-0012 (JP)
- UNO, Kakishi
Kawasaki-shi, Kanagawa 211-0012 (JP)

(74) Representative: Plasseraud IP
104 Rue de Richelieu
CS92104
75080 Paris Cedex 02 (FR)

# (54) ISOPOLYOXOTUNGSTATE SALT COMPOUND, SOLVATE THEREOF OR MIXTURE OF SAID COMPOUND AND SOLVATE, AND METHOD FOR PRODUCING SAID COMPOUND, SOLVATE OR MIXTURE

(57) [Object] To provide a novel isopolyoxotungstate compound or a solvate thereof or a mixture thereof and a method for producing them.

[Solution] An isopolyoxotungstate compound represented by the general formula (I) or a solvate thereof or a mixture thereof and a method for producing them.

$$(A^{m+})_{x/m}(B^{n+})_{y/n}(C^{-(x+y)}) \qquad (I)$$

EP 4 635 963 A1

## Description

### Technical Field

The present invention relates to an isopolyoxotungstate compound or a solvate thereof or a mixture thereof and a method for producing them.

### Background Art

A polyoxometalate (POM) is an anionic metal oxide cluster and has a structure in which an early transition metal with an electron configuration of $d^0$ or $d^1$ (for example, Mo (hexavalent or pentavalent), W (hexavalent or pentavalent), V (pentavalent), Nb (pentavalent), Ta (pentavalent), or the like) is oxygen-cross-linked.

Various structures can be adopted by incorporating various elements into the anionic metal oxide cluster of POM. Depending on the combination of constituent elements of POM, the structure thereof, and the like, physicochemical properties, such as oxidation-reduction ability, light absorption ability, and acidity, can be changed.

For example, Patent Literature 1 describes that a POM containing polyoxomolybdic acid and a compound with a pyridinium skeleton has a high photoluminescence quantum yield.

Polyoxotungstic acid (POT), which is one type of POM, includes an isopolyoxotungstic acid composed of an oxygen acid containing tungsten and a heteropolyoxotungstic acid composed of an oxygen acid containing tungsten and one or more heteroatoms other than tungsten. The heteroatom constituting the heteropolyoxotungstic acid can be selected from a large number of Groups I to VIII elements, such as Si, Ge, and P, and POMs with different physicochemical properties can be formed by changing the combination, constituent ratio, and the like thereof.

Non-patent Literature 1 describes that $[W_4O_{16}]^{8-}$, $[W_6O_{19}]^{2-}$, $[W_7O_{24}]^{6-}$, $[H_3W_6O_{22}]^{5-}$, $[W_{10}O_{32}]^{4-}$, $[H_2W_{12}O_{42}]^{10-}$, $[H_2W_{12}O_{40}]^{6-}$, $[H_4W_{19}O_{62}]^{6-}$, $[H_4W_{22}O_{74}]^{12-}$ $[W_{24}O_{84}]^{24-}$, $[H_{10}W_{34}O_{116}]^{18-}$, $[H_{12}W_{36}O_{120}]^{12-}$, $[H_{12}W_{48}O_{164}]^{28-}$, $[H_{20}W_{56}O_{190}]^{24-}$, and the like are reported as isopolyoxotungstic acids. Furthermore, Non-patent Literature 1 points out that the isopolyoxotungstic acid can be a useful building block or precursor for constructing a novel anionic metal oxide cluster and that the search, design, and the like of a derivative based on the isopolyoxotungstic acid are important research subjects in the fields of catalysts, magnetic materials, pharmaceuticals, optical physics, and the like.

### Citation List

### Patent Literature

PTL 1: U.S. Patent No. 11192908

### Non Patent Literature

NPL 1: Ya-Jie Liu et al., "Recent advances in isopolyoxotungstates and their derivatives", Acta Crystallographica, C74, 1202-1221 (2018).

## Summary of Invention

### Technical Problem

In view of the above circumstances, an object of the present invention is to provide a novel isopolyoxotungstate compound or a solvate thereof or a mixture thereof and a method for producing them.

Solution to Problem

As a result of intensive studies to solve the above problems, the present inventors have succeeded in synthesizing an isopolyoxotungstate compound represented by the general formula (I) or a solvate thereof or a mixture thereof and have completed the present invention by finding a method for efficiently synthesizing an isopolyoxotungstate compound represented by the general formula (I) or a solvate thereof or a mixture thereof.

More specifically, the present invention relates to the following inventions.

<1> An isopolyoxotungstate compound represented by the general formula (I) or a solvate thereof:

$$(A^{m+})_{x/m}(B^{n+})_{y/n}(C^{-(x+y)}) \qquad (I)$$

wherein

$A^{m+}$ each independently denotes $H^+$, a metal ion, or an ammonium ion;
$B^{n+}$ each independently denotes a sulfonium cation or an iodonium cation;
$C^{-(x+y)}$ denotes an isopolyoxotungstic acid; and
m denotes an integer in the range of 1 to 5, n denotes an integer of 1 or 2, x and x/m denote an integer, and y and y/n denote a natural number.

<2> The isopolyoxotungstate compound or a solvate thereof according to <1>, wherein the isopolyoxotungstic acid is $[W_4O_{13}]^{2-}$, $[W_5O_{16}]^{2-}$, $[W_6O_{19}]^{2-}$, $[W_7O_{22}]^{2-}$, $[W_7O_{24}]^{6-}$, $[H_zW_{12}O_{40}]^{-(8-z)}$ (wherein z denotes an integer in the range of 1 to 4), $[W_{10}O_{32}]^{4-}$, $[H_4W_{11}O_{38}]^{6-}$, $[H_7W_{11}O_{40}]^{7-}$, $[HW_5O_{19}]^{7-}$, $[H_3W_{11}O_{22}]^{5-}$, $[H_4W_{22}O_{74}]^{12-}$, or $[H_{10}W_{34}O_{116}]^{18-}$.
<3> An isopolyoxotungstate mixture comprising two or more different isopolyoxotungstate compounds or solvates thereof according to <1>, wherein the $B^{n+}$ denotes a sulfonium cation.
<4> The isopolyoxotungstate mixture according to <3>, wherein a mole ratio of S to W in XRF analysis or XPS analysis is S:W = 1 to 8:4 to 12.
<5> An isopolyoxotungstate mixture comprising two or more different isopolyoxotungstate compounds or solvates thereof according to <1>, wherein the $B^{n+}$ denotes an iodonium cation.
<6> The isopolyoxotungstate mixture according to <5>, wherein a mole ratio of I to W in XRF analysis or XPS analysis is I:W = 1 to 8:4 to 12.
<7> The isopolyoxotungstate compound or a solvate thereof according to <1>, wherein the sulfonium cation is a sulfonium cation represented by the general formula (II-1):

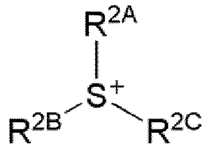
(II-1)

wherein

$R^{2A}$, $R^{2B}$, and $R^{2C}$ each independently denote an optionally substituted $C_{1-18}$ hydrocarbyl group, 3- to 18-membered non-aromatic heterocyclic group, or 5- to 18-membered aromatic heterocyclic group;
a divalent carbon atom at any position excluding a terminal of $R^{2A}$ to $R^{2C}$ may be replaced by -O-, -C(=O)-, COO-, -OCO-, -CONH-, -NHCO-, -S-, or $SO_2$- (provided that adjacent divalent carbon atoms are not replaced at the same time); and
any two of $R^{2A}$, $R^{2B}$, and $R^{2C}$ may be linked to each other directly by a single bond or via a divalent linking group -O-, -S-, -C(=O)-, -S(=O)-, $-S(=O)_2-$, -C(=O)O-, or a $C_{1-3}$ alkylene group to form a ring together with a sulfur atom in the formula (II-1), or
a sulfonium dication represented by the general formula (II-2):

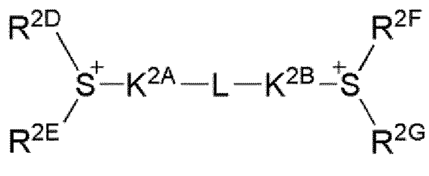
(II-2)

wherein
$R^{2D}$, $R^{2E}$, $R^{2F}$, and $R^{2G}$ each independently denote an optionally substituted $C_{1-18}$ hydrocarbyl group, 3- to 18-membered non-aromatic heterocyclic group, or 5- to 18-membered aromatic heterocyclic group;
a divalent carbon atom at any position excluding a terminal of $R^{2D}$, $R^{2E}$, $R^{2F}$, and $R^{2G}$ may be replaced by -O-, -C(=O)-, COO-, -OCO-, -CONH-, -NHCO-, -S-, or $SO_2$-(provided that adjacent divalent carbon atoms are not replaced at the same time);
$K^{2A}$, $K^{2B}$, and L each independently denote an optionally substituted $C_{1-18}$ hydrocarbylene group;
a divalent carbon atom at any position of $K^{2A}$, $K^{2B}$, and L may be replaced by - O-, -C(=O)-, COO-, -OCO-, -CONH-, -NHCO-, -S-, or $SO_2$- (provided that adjacent divalent carbon atoms are not replaced at the same time);

and

any two of $R^{2D}$, $R^{2E}$, and $K^{2A}$ and/or any two of $R^{2F}$, $R^{2G}$, and $K^{2B}$ may be linked to each other directly by a single bond or via a divalent linking group -O-, -S-, -C(=O)-, - S(=O)-, -S(=O)$_2$-, -C(=O)O-, or a $C_{1-3}$ alkylene group to form a ring together with a sulfur atom in the formula (II-2).

<8> The isopolyoxotungstate compound or a solvate thereof according to <1>, wherein the sulfonium cation is a sulfonium cation represented by the general formula (III-I), (III-2), (III-3), (III-4), or (III-5),

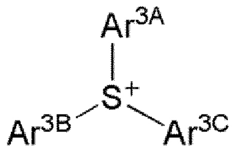

(III-1)

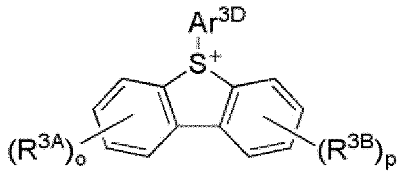

(III-2)

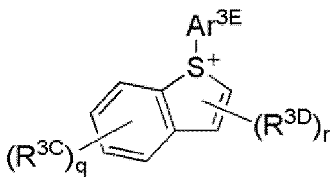

(III-3)

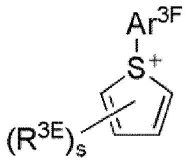

(III-4)

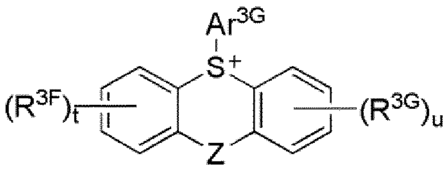

(III-5)

in the formulae (III-I) to (III-5),

$Ar^{3A}$, Ar3B, $Ar^{3C}$, $Ar^{3D}$, $Ar^{3E}$, $Ar^{3F}$, and $Ar^{3G}$ each independently denote an optionally substituted $C_{6-18}$ aryl group or 5- to 18-membered heterocyclic group,

in the optionally substituted $C_{6-18}$ aryl group or 5- to 18-membered heterocyclic group, at least part of hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of hydrogen atoms may be substituted by a halogen atom, a

hydroxy group, a thiol group, a nitro group, a cyano group, a carboxy group, an amino group, or a sulfo group, a divalent carbon atom at any position excluding a terminal of the substituent may be replaced by -O-, -C(=O)-, COO-, -OCO-, -CONH-, -NHCO-, -S-, or $SO_2$-(provided that adjacent divalent carbon atoms are not replaced at the same time);

$R^{3A}$, $R^{3B}$, $R^{3C}$, $R^{3D}$, $R^{3E}$, $R^{3F}$, and $R^{3G}$ each independently denote a halogen atom, a hydroxy group, a thiol group, a nitro group, a cyano group, a carboxy group, an amino group, or a sulfo group, or each independently denote a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of hydrogen atoms may be substituted by a halogen atom, a hydroxy group, a thiol group, a nitro group, a cyano group, a carboxy group, an amino group, or a sulfo group,

the divalent carbon atom at any position excluding the terminal may be replaced by -O-, -C(=O)-, COO-, -OCO-, -CONH-, -NHCO-, -S-, or $SO_2$- (provided that adjacent divalent carbon atoms are not replaced at the same time);

o, p, q, s, t, and u each denote an integer in the range of 0 to 4, r denotes an integer in the range of 0 to 2, and when o to u denote an integer of 2 or more, $R^{3D}$, $R^{3E}$, $R^{3F}$, $R^{3G}$, $R^{3H}$, $R^{3I}$, and $R^{3J}$ may be the same or different; and

Z denotes -O-, -S-, -C(=O)-, -S(=O)-, -S(=O)$_2$-, -C(=O)O-, or a $C_{1-3}$ alkylene group.

<9> The isopolyoxotungstate compound or a solvate thereof according to <1>, wherein the iodonium cation is an iodonium cation represented by the general formula (IV):

$$R^{4A}—I^{+}—R^{4B} \qquad (IV)$$

wherein

$R^{4A}$ and $R^{4B}$ each independently denote an optionally substituted $C_{1-18}$ hydrocarbyl group, an optionally substituted 3- to 18-membered non-aromatic heterocyclic group, or an optionally substituted 5- to 18-membered aromatic heterocyclic group, and

$R^{4A}$ and $R^{4B}$ may be linked to each other directly by a single bond or via a divalent linking group -O-, -S-, -C(=O)-, -S(=O)-, -S(=O)$_2$-, -C(=O)O-, or a $C_{1-3}$ alkylene group to form a ring together with an iodine atom in the formula (V-1).

<10> 10. The isopolyoxotungstate compound or a solvate thereof according to <1>, wherein the iodonium cation is an iodonium cation represented by the general formula (V-1):

$$Ar^{5A}—I^{+}—Ar^{5B} \qquad (V-1)$$

wherein

$Ar^{5A}$ and $Ar^{5B}$ each independently denote an optionally substituted $C_{6-18}$ aryl group or 5- to 18-membered heterocyclic group,

in the optionally substituted $C_{6-18}$ aryl group or 5- to 18-membered heterocyclic group, at least part of hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of hydrogen atoms may be substituted by a halogen atom, a hydroxy group, a thiol group, a nitro group, a cyano group, a carboxy group, an amino group, or a sulfo group,

a divalent carbon atom at any position excluding a terminal of the substituent may be replaced by -O-, -C(=O)-, -COO-, -OCO-, -CONH-, -NHCO-, -S-, or $-SO_2-$(provided that adjacent divalent carbon atoms are not replaced at the same time); and

$Ar^{5A}$ and $Ar^{5B}$ may be linked to each other directly by a single bond or via a divalent linking group -O-, -S-, -C(=O)-, -S(=O)-, $-S(=O)_2-$, -C(=O)O-, or a $C_{1-3}$ alkylene group to form a ring together with an iodine atom in the formula (V-1); or

(V-2):

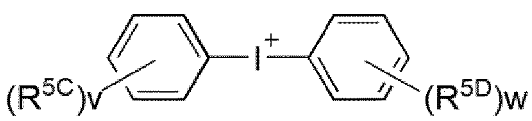

(V − 2)

wherein

$R^{5C}$ and $R^{5D}$ each independently denote a halogen atom, a hydroxy group, a thiol group, a nitro group, a cyano group, a carboxy group, an amino group, or a sulfo group, or each independently denote a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of hydrogen atoms may be substituted by a halogen atom, a hydroxy group, a thiol group, a nitro group, a cyano group, a carboxy group, an amino group, or a sulfo group,

the divalent carbon atom at any position excluding the terminal may be replaced by -O-, -C(=O)-, -COO-, -OCO-, -CONH-, -NHCO-, -S-, or $-SO_2-$ (provided that adjacent divalent carbon atoms are not replaced at the same time); and

v and w each denote an integer in the range of 0 to 5, and when v and w denote an integer of 2 or more, $R^{5C}$ and $R^{5D}$ may be the same or different.

<11> A method for producing an isopolyoxotungstate compound represented by the general formula (I) or a solvate thereof, the method comprising:

reacting a compound represented by the general formula (VI-1) with a compound represented by the general formula (VI-2) to perform salt exchange;

$$(A^{m+})_{x/m}(B^{n+})_{y/n}(C^{-(x+y)}) \qquad (I)$$

$$(A^{m+})_{x/m}(A'^{m'+})_{y/m'}(C^{-(x+y)}) \qquad (VI\text{-}1)$$

$$(B^{n+})_{y/n}(Y^{-})_{y} \qquad (VI\text{-}2)$$

wherein

$A^{m+}$ each independently denotes $H^+$, a metal ion, or an ammonium ion;

$A'^{m'+}$ each independently denotes a metal ion or an ammonium ion;

$B^{n+}$ each independently denotes a sulfonium cation or an iodonium cation;

$C^{-(x+y)}$ denotes an isopolyoxotungstic acid;

$Y^-$ denotes a monovalent counter anion; and

m and m' denote an integer in the range of 1 to 5, n denotes an integer of 1 or 2, x and x/m denote an integer, and y/m', y, and y/n denote a natural number.

<12> A method for producing an isopolyoxotungstate compound represented by the general formula (I) or a solvate thereof, the method comprising:

reacting a compound represented by the general formula (VI-1) with a compound represented by the general formula (VI-2) to perform salt exchange; and

removing a compound represented by the general formula (VI-3) from the resulting compounds represented by the general formulae (I) and (VI-3);

$$(A^{m+})_{x/m}(B^{n+})_{y/n}(C^{-(x+y)}) \qquad (I)$$

$$(A^{m+})_{x/m}(A'^{m'+})_{y/m'}(C^{-(x+y)}) \qquad (VI-1)$$

$$(B^{n+})_{y/n}(Y^{-})_{y} \qquad (VI-2)$$

$$(A'^{m'+})_{y/m'}(Y^{-})_{y} \qquad (VI-3)$$

wherein
$A^{m+}$ each independently denotes $H^+$, a metal ion, or an ammonium ion;
$A'^{m'+}$ each independently denotes a metal ion or an ammonium ion;
$B^{n+}$ each independently denotes a sulfonium cation or an iodonium cation;
$C^{-(x+y)}$ denotes an isopolyoxotungstic acid;
$Y^-$ denotes a monovalent counter anion; and
m and m' denote an integer in the range of 1 to 5, n denotes an integer of 1 or 2, x and x/m denote an integer, and y/m', y, and y/n denote a natural number.

<13> A method for producing an isopolyoxotungstate compound represented by the general formula (I) or a solvate thereof, the method comprising:

ion-exchanging $A'^{m'+}$ of a compound represented by the general formula (VI-1) with $H^+$ using an ion-exchange resin; and
reacting the resulting compound represented by the general formula (VI-4) with a compound represented by the general formula (VI-2);

$$(A^{m+})_{x/m}(B^{n+})_{y/n}(C^{-(x+y)}) \qquad (I)$$

$$(A^{m+})_{x/m}(A'^{m'+})_{y/m'}(C^{-(x+y)}) \qquad (VI-1)$$

$$(A^{m+})_{x/m}(H^{+})_{y}(C^{-(x+y)} \qquad (VI-4)$$

$$(B^{n+})_{y/n}(Y^{-})_{y} \qquad (VI-2)$$

wherein
$A^{m+}$ each independently denotes $H^+$, a metal ion, or an ammonium ion;
$A'^{m'+}$ each independently denotes a metal ion or an ammonium ion;
$B^{n+}$ each independently denotes a sulfonium cation or an iodonium cation;
$C^{-(x+y)}$ denotes an isopolyoxotungstic acid;
$Y^-$ denotes a monovalent counter anion; and

m and m' denote an integer in the range of 1 to 5, n denotes an integer of 1 or 2, x and x/m denote an integer, and y/m', y, and y/n denote a natural number.

### Advantageous Effects of Invention

The present invention can provide a novel isopolyoxotungstate compound or a solvate thereof or a mixture thereof and a method for producing them.

### Description of Embodiments

Preferred embodiments of the present invention will be described in detail below. However, the present invention is not limited to the following embodiments.

[1. Isopolyoxotungstate Compound or Solvate Thereof or Mixture Thereof]

An isopolyoxotungstate compound according to an embodiment of the present invention is represented by the following general formula (I):

$$(A^{m+})_{x/m}(B^{n+})_{y/n}(C^{-(x+y)}) \qquad (I)$$

[wherein

$A^{m+}$ each independently denotes $H^+$, a metal ion, or an ammonium ion;
$B^{n+}$ each independently denotes a sulfonium cation or an iodonium cation;
$C^{-(x+y)}$ denotes an isopolyoxotungstic acid; and
m denotes an integer in the range of 1 to 5, n denotes an integer of 1 or 2, x and x/m denote an integer, and y and y/n denote a natural number.]
$A^{m+}$ is hereinafter also referred to as "another counter cation".

"Isopolyoxotungstic Acid", "Sulfonium Cation and Iodonium Cation", "Another Counter Cation", "Compound or Solvate or Mixture Thereof", and "Solvate" will be sequentially described.

[1-1. Isopolyoxotungstic Acid]

An isopolyoxotungstate compound according to the present embodiment is represented by the following general formula (I):

$$(A^{m+})_{x/m}(B^{n+})_{y/n}(C^{-(x+y)}) \qquad (I)$$

$C^{-(x+y)}$ denotes an isopolyoxotungstic acid; and
x denotes an integer, and y denotes a natural number.

An isopolyoxotungstic acid according to the present embodiment is not particularly limited, and a known and commonly used isopolyoxotungstic acid can be used. The isopolyoxotungstic acid is, for example, $[W_4O_{13}]^{2-}$, $[W_4O_{16}]^{8-}$, $[W_5O_{16}]^{2-}$, $[W_6O_{19}]^{2-}$, $[W_7O_{22}]^{2-}$, $[W_7O_{24}]^{6-}$, $[H_3W_6O_{22}]^{5-}$, $[W_{10}O_{32}]^{4-}$, $[H_2W_{12}O_{42}]^{10-}$, $[H_1W_{12}O_{40}]^{7-}$, $[H_2W_{12}O_{40}]^{6-}$, $[H_3W_{12}O_{40}]^{5-}$, $[H_4W_{12}O_{40}]^{4-}$, $[H_4W_{11}O_{38}]^{6-}$, $[H_7W_{11}O_{40}]^{7-}$, $[HW_5O_{19}]^{7-}$, $[H_3W_{11}O_{22}]^{5-}$, $[H_4W_{19}O_{62}]^{6-}$, $[H_4W_{22}O_{74}]^{12-}$, $[W_{24}O_{84}]^{24-}$, $[H_{10}W_{34}O_{116}]^{18-}$, $[H_{12}W_{36}O_{120}]^{12-}$, $[H_{12}W_{48}O_{164}]^{28-}$, $[H_{20}W_{56}O_{190}]^{24-}$, or the like. The number of hydrogen atoms in the isopolyoxotungstic acid is not limited to the numbers described above.

From the perspective of high thermal stability and stability in an oxidizing atmosphere, the isopolyoxotungstic acid according to the present embodiment is preferably $[W_4O_{13}]^{2-}$, $[W_5O_{16}]^{2-}$, $[W_6O_{19}]^{2-}$, $[W_7O_{22}]^{2-}$, $[W_7O_{24}]^{6-}$, $[W_{10}O_{32}]^{4-}$, $[H_zW_{12}O_{40}]^{-(8-z)}$ (wherein z denotes an integer in the range of 1 to 4), $[H_4W_{11}O_{38}]^{6-}$, $[H_7W_{11}O_{40}]^{7-}$, $[HW_5O_{19}]^{7-}$, $[H_3W_{11}O_{22}]^{5-}$, $[H_4W_{22}O_{74}]^{12-}$, or $[H_{10}W_{34}O_{116}]^{18-}$, more preferably $[W_6O_{19}]^{2-}$, $[W_7O_{24}]^{6-}$, $[W_{10}O_{32}]^{4-}$, $[H_zW_{12}O_{40}]^{-(8-z)}$ (wherein z denotes an integer in the range of 1 to 4), $[W_6O_{19}]^{2-}$, $[W_7O_{24}]^{6-}$, $[W_{10}O_{32}]^{4-}$, $[H_2W_{12}O_{40}]^{6-}$, or $[H_3W_{12}O_{40}]^{5-}$.

It should be noted that the isopolyoxotungstic acid may include all isomers, such as geometric isomers, optical isomers, rotational isomers, stereoisomers, and tautomers, which are structurally present, and isomer mixtures, and is not limited to the description of convenient chemical formulae.

For example, it is known that a Keggin-type isopolyoxotungstic acid ($[H_zW_{12}O_{40}]^{-(8-z)}$ (wherein z denotes an integer in the range of 1 to 4)) has five isomers of $\alpha$, $\beta$, $\gamma$, $\delta$, and $\varepsilon$ types depending on the manner of connection and arrangement of four $W_3O_{13}$ units. The chemical formula $[H_zW_{12}O_{40}]^{-(8-z)}$ (wherein z denotes an integer in the range of 1 to 4) includes these isomers or a mixture of these isomers.

[1-2. Sulfonium Cation and Iodonium Cation]

An isopolyoxotungstate compound according to the present embodiment is represented by the following general formula (I):

$$(A^{m+})_{x/m}(B^{n+})_{y/n}(C^{-(x+y)}) \qquad (I)$$

$B^{n+}$ each independently denotes a sulfonium cation or an iodonium cation; and
n denotes an integer of 1 or 2, and y and y/n denote a natural number.

Meanings of terms, symbols, and the like used in the present description will be described below, and embodiments of the present invention will be described in more detail below.

The term "halogen atom", as used herein, refers to a fluorine atom, a chlorine atom, a bromine atom, or an iodine atom.

In the present description, for example, the term "$C_{1-6}$" or the like means the number of carbon atoms of a group serving as a mother nucleus.

The term "$C_{1-18}$ hydrocarbylene group", as used herein, refers to a divalent hydrocarbon group formed by removing two hydrogen atoms from a hydrocarbon with 1 to 18 carbon atoms. The hydrocarbylene group may be linear, branched, or partially or fully cyclic. Examples of the hydrocarbylene group include an alkylene group and an arylene group.

A divalent carbon atom at any position of the "$C_{1-18}$ hydrocarbylene group" may be replaced by -O-, -S-, -C(=O)-, -COO-, -OCO-, -CONH-, -NHCO-, -S-, -SO-, or -$SO_2$-(provided that adjacent divalent carbon atoms are not replaced at the same time).

The "$C_{1-18}$ hydrocarbylene group" is, for example, but not limited to, a "$C_{1-18}$ alkylene group", such as a methylene group, an ethylene group, a n-propylene group, an i-propylene group, a cyclopentadiyl group, a cyclohexanediyl group, an oxyethane-1,1-diyl group, an oxyethane-1,2-diyl group, an oxypropane-1,3-diyl group, an oxypropane-1,2-diyl group, a 2-methylpropane-1,3-diyl group, or an oxyethyleneoxyethane-1,1-diyl group; a "$C_{6-18}$ arylene group", such as a 1,4-phenylene group, a 1,3-phenylene group, a 1,2-phenylene group, a 1,4-naphthylene group, a 1,5-naphthylene group, a 1,8-naphthylene group, a 4,4'-biphenylene group, an anthracenediyl group, a phenanthrenediyl group, a naphthacenediyl group, a pyrenediyl group, a perylenediyl group, or a chrysenediyl group; or the like.

The term "$C_{1-18}$ alkyl group", as used herein, refers to a linear or branched alkyl group with 1 to 18 carbon atoms.

Furthermore, a divalent carbon atom at any position excluding the terminals contained in the "$C_{1-18}$ alkyl group" may be replaced by -O-, -C(=O)-, COO-, -OCO-, - CONH-, -NHCO-, -S-, or $SO_2$-. However, adjacent divalent carbon atoms are not replaced at the same time.

The "$C_{1-18}$ alkyl group" is, for example, but not limited to, a methyl group, an ethyl group, a n-propyl group, an i-propyl group, a n-butyl group, an i-butyl group, a sec-butyl group, a t-butyl group, a n-pentyl group, an i-pentyl group, a sec-pentyl group, a t-pentyl group, a neopentyl group, a 1-methylbutyl group, a 2-methylbutyl group, a 1,1-dimethylpropyl group, a 1,2-dimethylpropyl group, a n-hexyl group, an i-hexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a n-heptyl group, a n-octyl group, a n-nonyl group, a n-decyl group, a n-undecyl group, a n-dodecyl group, or the like.

The "$C_{1-18}$ alkyl group" in which a divalent carbon atom at any position excluding the terminals thereof is replaced by -O-, -C(=O)-, COO-, -OCO-, -CONH-, -NHCO-, -S-, or $SO_2$- is, for example, but not limited to, a 2-methoxyethoxymethyl group, an ethoxycarbonylmethyl group, or the like.

The term "$C_{1-18}$ haloalkyl group", as used herein, refers to a group in which one or more hydrogen atoms of the "$C_{1-18}$ alkyl group" are substituted by a halogen atom.

The "$C_{1-18}$ haloalkyl group" is, for example, but not limited to, a dichloromethyl group, a trifluoromethyl group, a 2,2-difluoroethyl group, a 2,2,2-trifluoroethyl group, a pentafluoroethyl group, a 3,3,3-trifluoropropyl group, or the like.

The term "$C_{2-18}$ alkenyl group", as used herein, refers to an alkenyl group with 2 or more carbon atoms having one or more double bonds in the "$C_{1-18}$ alkyl group" and includes an alkadienyl group, an alkatrienyl group, and the like.

The "$C_{2-18}$ alkenyl group" is, for example, but not limited to, a vinyl group (ethenyl group), an allyl group (2-propenyl group), a 1-propenyl group, an isopropenyl group (1-methyl vinyl group), a 1-butenyl group, a 2-butenyl group, a 3-butenyl group, a pentenyl group, a hexenyl group, a heptenyl group, an octenyl group, a nonenyl group, a decenyl group, an undecenyl group, a dodecenyl group, or the like.

The term "$C_{2-18}$ alkynyl group", as used herein, refers to an alkynyl group with 2 or more carbon atoms having one or more triple bonds in the "$C_{1-18}$ alkyl group".

The "$C_{2-18}$ alkynyl group" is, for example, but not limited to, an ethynyl group, a 1-propynyl group, a 2-propynyl group, a pentynyl group, a hexynyl group, a heptynyl group, an octynyl group, a nonynyl group, a decynyl group, an undecynyl group, a dodecynyl group, or the like.

The term "$C_{3-18}$ alicyclic group", as used herein, refers to a hydrocarbon group with 3 to 18 carbon atoms and with a monocyclic or polycyclic structure in whole or in part. The alicyclic group includes a cycloalkyl group, a cycloalkenyl group, a cycloalkynyl group, a monocycloalkyl group, a polycycloalkyl group, and the like.

A divalent carbon atom at any position excluding the terminals contained in the "$C_{3-18}$ alicyclic group" may be replaced by -O-, -C(=O)-, COO-, -OCO-, -CONH-, - NHCO-, -S-, or $SO_2$- (provided that adjacent divalent carbon atoms are not replaced at the same time).

The "$C_{3-18}$ cycloalkyl group" is, for example, but not limited to, a cyclopropyl group, a cyclobutyl group, a cyclopentyl group, a 1-i-propylcyclopentane-1-yl group, a cyclohexyl group, a t-butylcyclohexyl group, a tricyclodecanyl

group, a cycloheptyl group, a cyclooctyl group, a cyclodecyl group, a 2-methyladamantane-2-yl group, a 2-i-propyladamantane-2-yl group, a bornyl group, a norbonyl group, a fenchyl group, a pinanyl group, an adamantyl group, a tricyclodecyl group, a tetracyclododecyl group, a cyclopropylmethyl group, a cyclobutylmethyl group, a cyclopentylmethyl group, a cyclohexylmethyl group, a bornylmethyl group, a norbornylmethyl group, an adamantylmethyl group, a 1-methylcyclopentyloxycarbonylmethyl group, a cyclopentenyl group, a cyclohexenyl group, a cycloheptenyl group, or the like.

The term "3- to 18-membered non-aromatic heterocyclic group", as used herein, refers to a 3- to 18-membered non-aromatic heterocyclic group containing one or more heteroatoms selected from the group consisting of a nitrogen atom, an oxygen atom, and a sulfur atom, may be monocyclic, polycyclic, or condensed, and may be saturated or partially unsaturated.

The "3- to 18-membered non-aromatic heterocyclic group" is, for example, but not limited to, an aziridinyl group, an azetidil group, a pyrrolidinyl group, a pyrrolyl group, a piperidinyl group, a piperazinyl group, a morpholinyl group, a thiomorpholinyl group, a tetrahydrofuryl group, a tetrahydropyranyl group, an oxetanyl group, a tetrahydrofuryl group, a tetrahydropyranyl group, an imidazolinyl group, an oxazolinyl group, a 2,5-diazabicyclo[2.2.1]heptyl group, a 2,5-diazabicyclo[2.2.2]octyl group, a 3,8-diazabicyclo[3.2.1]octyl group, a 1,4-diazabicyclo[4.3.0]nonyl group, a 1-azaadamantyl group, a 2-azaadamantyl group, or the like.

The term "$C_{6-18}$ aryl group", as used herein, refers to an aromatic hydrocarbon ring group with 6 to 18 carbon atoms and may be a monocyclic, polycyclic, or fused ring.

The "$C_{6-18}$ aryl group" is, for example, but not limited to, a phenyl group, a **1-**naphthyl group, a 2-naphthyl group, an azulenyl group, a pentalenyl group, a heptalenyl group, an indacenyl group, an acenaphthyl group, a phenanthrenyl group, an anthracenyl group, or the like.

The term "$C_{7-18}$ aralkyl group", as used herein, refers to a group in which a substitutable portion of a "$C_{1-12}$ alkyl group" is substituted by the "$C_{6-12}$ aryl group".

The "$C_{7-18}$ aralkyl group" is, for example, but not limited to, a benzyl group, a phenethyl group, a 3-phenylpropyl group, a 4-phenylbutyl group, a 1-naphthylmethyl group, or a 2-naphthylmethyl group.

The term "5- to 18-membered aromatic heterocyclic group", as used herein, refers to a monocyclic, polycyclic, or condensed 5- to 18-membered aromatic heterocyclic group containing one or more heteroatoms selected from the group consisting of a nitrogen atom, an oxygen atom, and an oxygen atom.

The "5- to 18-membered aromatic heterocyclic group" is, for example, but not limited to, a pyrrolyl group, a furyl group, a thienyl group, an imidazolyl group, a triazolyl group, a tetrazolyl group, a pyrazolyl group, an oxazolyl group, an isoxazolyl group, a thiazolyl group, an isothiazolyl group, furazanyl group, a thiadiazolyl group, an oxadiazolyl group, a pyridyl group, a pyrazinyl group, a pyridazinyl group, a pyrimidinyl group, a triazinyl group, a purinyl group, a pteridinyl group, a quinolyl group, an isoquinolyl group, a naphthyridinyl group, a quinoxalinyl group, a cinnolinyl group, a quinazolinyl group, a phthalazinyl group, an imidazopyridyl group, an imidazothiazolyl group, an imidazooxazolyl group, a benzothiazolyl group, a benzoxazolyl group, a benzimidazolyl group, an indolyl group, an isoindolyl group, an indazolyl group, a pyrrolopyridyl group, a thienopyridyl group, a furopyridyl group, a benzothiadiazolyl group, a benzoxadiazolyl group, a pyridopyrimidinyl group, a benzofuryl group, a benzothienyl group, or the like.

The term "$C_{1-18}$ hydrocarbyl group", as used herein, refers to a monovalent group formed by removing one hydrogen atom from a hydrocarbon with 1 to 18 carbon atoms. Examples of the hydrocarbyl group include an alkyl group, an alkenyl group, an alkynyl group, an alicyclic group, an aryl group, an aralkyl group, and the like.

A divalent carbon atom at any position excluding the terminals contained in the "$C_{1-18}$ hydrocarbyl group" may be replaced by -O-, -C(=O)-, COO-, -OCO-, -CONH-, - NHCO-, -S-, or $SO_2$- (provided that adjacent divalent carbon atoms are not replaced at the same time).

The "$C_{1-18}$ hydrocarbyl group" is, for example, but not limited to, a "$C_{1-18}$ alkyl group", a "$C_{2-18}$ alkenyl group", a "$C_{2-18}$ alkynyl group", a "$C_{3-18}$ alicyclic group", a "$C_{6-18}$ aryl group", a "$C_{7-18}$ aralkyl group", or the like.

The term "$C_{1-18}$ hydrocarbyloxy group", as used herein, refers to a group in which an oxygen atom (-O-) is bonded to the "$C_{1-18}$ hydrocarbyl group".

The "$C_{1-18}$ hydrocarbyloxy group" is, for example, but not limited to, a "$C_{1-18}$ alkoxy group", such as a methoxy group, an ethoxy group, a n-propoxy group, an i-propoxy group, a n-butoxy group, an i-butoxy group, a sec-butoxy group, a t-butoxy group, a n-pentoxy group, an i-pentoxy group, a sec-pentoxy group, a n-hexoxy group, an i-hexoxy group, a 1,1-dimethylpropyloxy group, a 1,2-dimethylpropyloxy group, a 2,2-dimethylpropyloxy group, a 1-methyl-2-ethylpropyloxy group, a 1-ethyl-2-methylpropyloxy group, a 1,1,2-trimethylpropyloxy group, a 1,2,2-trimethylpropyloxy group, a 1,1-dimethylbutyloxy group, a 1,2-dimethylbutyloxy group, a 2,2-dimethylbutyloxy group, a 2,3-dimethylbutyloxy group, a 1,3-dimethylbutyloxy group, a 2-ethylbutyloxy group, a 2-methylpentyloxy group, or a 3-methylpentyloxy group; a "$C_{3-18}$ alicyclic oxy group", such as a cyclopropyloxy group, a cyclobutyloxy group, a cyclopentyloxy group, a cyclohexyloxy group, a cycloheptyloxy group, a cyclooctyloxy group, a 1-methylcyclopentyloxycarbonylmethoxy group, a 1-ethylcyclohexyloxycarbonylmethoxy group, or a 1-methyladamantyloxycarbonylmethoxy group; a "$C_{6-18}$ aryloxy group", such as a phenyloxy group, a 1-naphthyloxy group, a 2-naphthyloxy group, an azulenyloxy group, a pentalenyloxy group, a

heptalenyloxy group, an indacenyloxy group, an acenaphthyloxy group, a phenanthrenyloxy group, or an anthracenyloxy group; or the like.

The term "$C_{1-18}$ hydrocarbylcarbonyl group", as used herein, refers to a group in which a carbonyl group (-C(=O)-) is bonded to the "$C_{1-18}$ hydrocarbyl group".

The "$C_{1-18}$ hydrocarbylcarbonyl group" is, for example, but not limited to, a "$C_{1-18}$ alkyl carbonyl group", such as an acetyl group, a propionyl group, an isopropionyl group, a butyryl group, an isobutyryl group, a valeryl group, an isovaleryl group, a pentanoyl group, a 3-methylbutanoyl group, a pivaloyl group, a hexanoyl group, or a heptanoyl group; a "$C_{3-18}$ alicyclic carbonyl group", such as a cyclopropyl carbonyl group, a cyclobutyl carbonyl group, a cyclopentyl carbonyl group, a 2-methylcyclopentyl carbonyl group, a 3-methylcyclopentyl carbonyl group, a cyclohexyl carbonyl group, a 2-methylcyclohexyl carbonyl group, a 3-methylcyclohexyl carbonyl group, a 4-methylcyclohexyl carbonyl group, or an adamantyl carbonyl group; a "$C_{6-18}$ aryl carbonyl group", such as a benzoyl group, a 1-naphthoyl group, or a 2-naphthoyl group; or the like.

The term "$C_{1-18}$ hydrocarbylcarbonyloxy group", as used herein, refers to a group in which an oxygen atom (-O-) is bonded to the "$C_{1-18}$ hydrocarbylcarbonyl group".

The "$C_{1-18}$ hydrocarbylcarbonyloxy group" is, for example, but not limited to, a "$C_{1-18}$ alkyl carbonyloxy group", such as a methyl carbonyloxy group, an ethyl carbonyloxy group, a n-propyl carbonyloxy group, an isopropyl carbonyloxy group, a n-butyl carbonyloxy group, an isobutyl carbonyloxy group, a t-butyl carbonyloxy group, a n-pentyl carbonyloxy group, an isopentyl carbonyloxy group, or a hexyl carbonyloxy group; a "$C_{3-18}$ alicyclic carbonyloxy group", such as a cyclopropyl carbonyloxy group, a cyclobutyl carbonyloxy group, a cyclopentyl carbonyloxy group, or a cyclohexyl carbonyloxy group; a "$C_{6-18}$ aryl carbonyloxy group", such as a phenyl carbonyloxy group, a naphthyl carbonyloxy group, an acenaphthyl carbonyloxy group, a phenanthrenyl carbonyloxy group, or an anthracenyl carbonyloxy group; or the like.

The term "$C_{1-18}$ hydrocarbyloxycarbonyl group", as used herein, refers to a group in which a carbonyl group (-C(=O)-) is bonded to a "$C_{1-18}$ hydrocarbyloxy group".

The "$C_{1-18}$ hydrocarbyloxycarbonyl group" is, for example, but not limited to, a "$C_{1-18}$ alkoxycarbonyl group", such as a methoxycarbonyl group, an ethoxycarbonyl group, a n-propoxycarbonyl group, an i-propoxycarbonyl group, a n-butoxycarbonyl group, an i-butoxycarbonyl group, a sec-butoxycarbonyl group, a t-butoxycarbonyl group, a n-pentoxycarbonyl group, or a neopentyloxycarbonyl group; a "$C_{3-18}$ alicyclic oxycarbonyl group", such as a cyclopropyloxycarbonyl group, a cyclobutyloxycarbonyl group, a cyclopentyloxycarbonyl group, a cyclohexyloxycarbonyl group, a 2-methylcyclopentyloxycarbonyl group, a 3-methylcyclopentyloxycarbonyl group, a 2-methylcyclohexyloxycarbonyl group, a 3-methylcyclohexyloxycarbonyl group, or a 4-methylcyclohexyloxycarbonyl group; a "$C_{6-18}$ aryloxycarbonyl group", such as a phenoxycarbonyl group, a naphthoxycarbonyl group, an acenaphthyloxycarbonyl group, a phenanthrenyloxycarbonyl group, or an anthracenyloxycarbonyl group; or the like.

The term "$C_{1-18}$ hydrocarbyloxycarbonyloxy group", as used herein, refers to a group in which an oxygen atom (-O-) is bonded to the "$C_{1-18}$ hydrocarbyloxycarbonyl group".

The "$C_{1-18}$ hydrocarbyloxycarbonyloxy group" is, for example, but not limited to, a "$C_{1-18}$ alkoxycarbonyloxy group", such as a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a sec-butoxycarbonyloxy group, a t-butoxycarbonyloxy group, a n-pentyloxycarbonyloxy group, an i-pentyloxycarbonyloxy group, or a n-hexyloxycarbonyloxy group; a "$C_{3-18}$ alicyclic oxycarbonyloxy group", such as a cyclopropyloxycarbonyloxy group, a cyclobutyloxycarbonyloxy group, a cyclopentyloxycarbonyloxy group, or a cyclohexyloxycarbonyloxy group; a "$C_{6-18}$ aryloxycarbonyloxy group", such as a phenoxycarbonyloxy group, a naphthoxycarbonyloxy group, an acenaphthyloxycarbonyloxy group, a phenanthrenyloxycarbonyloxy group, or an anthracenyloxycarbonyloxy group; or the like.

The term "$C_{1-18}$ hydrocarbylamino group", as used herein, refers to a group in which one "$C_{1-18}$ hydrocarbyl group" is bonded to an amino group.

The "$C_{1-18}$ hydrocarbylamino group" is, for example, but not limited to, a "$C_{1-18}$ alkylamino group", such as a methylamino group, an ethylamino group, a n-propylamino group, an i-propylamino group, a n-butylamino group, an i-butylamino group, a sec-butylamino group, a t-butylamino group, a n-pentylamino group, an i-pentylamino group, a neopentylamino group, or a n-hexylamino group; a "$C_{3-18}$ alicyclic amino group", such as a cyclopropylamino group, a cyclobutylamino group, a cyclopentylamino group, a 2-methylcyclopentylamino group, a 3-methylcyclopentylamino group, a cyclohexylamino group, a 2-methylcyclohexylamino group, a 3-methylcyclohexylamino group, or a 4-methylcyclohexylamino group; a "$C_{6-18}$ arylamino group", such as a phenylamino group, a 1-naphthylamino group, or a 2-naphthylamino group; or the like.

The term "di-$C_{1-18}$ hydrocarbylamino group", as used herein, refers to a group in which two identical or different "$C_{1-18}$ hydrocarbyl groups" are bonded to an amino group.

The "di-$C_{1-18}$ hydrocarbylamino group" is, for example, but not limited to, a "di-$C_{1-18}$ alkylamino group", such as a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, or an N-ethyl-N-n-hexylamino group; a "di-$C_{3-18}$ alicyclic amino group", such as a dicyclopropylamino group, a dicyclobutylamino group, a dicyclopentylamino group, or a dicyclohexylamino group; a "di-$C_{6-18}$ arylamino group", such as a diphenylamino group or a phenylnaphthylamino group; an "N-$C_{1-18}$ alkyl-N-$C_{3-18}$ cycloalkylamino group", such as an N-methylcyclopentaneamino group or an N-methylcyclohexylamino group; an "N-$C_{1-18}$ alkyl-N-$C_{6-18}$ arylamino group", such as an N-methyl-2-phenylethylamino group or an N-ethyl-N-(4-methylphenyl)amino group; or the like.

The term "$C_{1-18}$ hydrocarbylaminocarbonyl group", as used herein, refers to a group in which a carbonyl group (-C(=O)-) is bonded to the "$C_{1-18}$ hydrocarbylamino group".

The "$C_{1-18}$ hydrocarbylaminocarbonyl group" is, for example, but not limited to, a "$C_{1-18}$ alkylaminocarbonyl group", such as a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, a n-pentylaminocarbonyl group, a 2-pentylaminocarbonyl group, a neopentylaminocarbonyl group, a 4-methyl-2-pentylaminocarbonyl group, a n-hexylaminocarbonyl group, or a 3-methyl-n-pentylaminocarbonyl group; a "$C_{3-18}$ alicyclic aminocarbonyl group", such as a cyclopropylaminocarbonyl group, a cyclobutylaminocarbonyl group, a cyclopentylaminocarbonyl group, a cyclohexylaminocarbonyl group, a 2-methylcyclopentylaminocarbonyl group, a 3-methylcyclopentylaminocarbonyl group, a 2-methylcyclohexylaminocarbonyl group, a 3-methylcyclohexylaminocarbonyl group, or a 4-methylcyclohexylaminocarbonyl group; or a "$C_{6-18}$ arylaminocarbonyl group", such as a phenylaminocarbonyl group, a 1-naphthylaminocarbonyl group, or a 2-naphthylaminocarbonyl group.

The term "di-$C_{1-18}$ hydrocarbylaminocarbonyl group", as used herein, refers to a group in which a carbonyl group (-C(=O)-) is bonded to a "di-$C_{1-18}$ hydrocarbylamino group".

The "di-$C_{1-18}$ hydrocarbylaminocarbonyl group" is, for example, but not limited to, a "di-$C_{1-18}$ alkylamino group", such as a dimethylaminocarbonyl group, a diethylaminocarbonyl group, a di-n-propylaminocarbonyl group, a diisopropylaminocarbonyl group, a di-n-butylaminocarbonyl group, a diisobutylaminocarbonyl group, a di-t-butylaminocarbonyl group, a di-n-pentylaminocarbonyl group, a di-n-hexylaminocarbonyl group, an N-ethyl-N-methylaminocarbonyl group, an N-methyl-N-n-propylaminocarbonyl group, an N-isopropyl-N-methylaminocarbonyl group, an N-n-butyl-N-methylaminocarbonyl group, an N-isobutyl-N-methylaminocarbonyl group, an N-t-butyl-N-methylaminocarbonyl group, an N-methyl-N-n-pentylaminocarbonyl group, an N-n-hexyl-N-methylaminocarbonyl group, an N-ethyl-N-n-propylaminocarbonyl group, an N-ethyl-N-isopropylaminocarbonyl group, an N-n-butyl-N-ethylaminocarbonyl group, an N-ethyl-N-isobutylaminocarbonyl group, an N-t-butyl-N-ethylaminocarbonyl group, an N-ethyl-N-n-pentylaminocarbonyl group, or an N-ethyl-N-n-hexylaminocarbonyl group; a "di-$C_{3-18}$ alicyclic aminocarbonyl group", such as a dicyclopropylaminocarbonyl group, a dicyclobutylaminocarbonyl group, a dicyclopentylaminocarbonyl group, or a dicyclohexylaminocarbonyl group; a "di-$C_{6-18}$ arylaminocarbonyl group", such as a diphenylaminocarbonyl group or a phenylnaphthylaminocarbonyl group; or the like.

The term "$C_{1-18}$ hydrocarbylcarbonylamino group", as used herein, refers to a group in which an amino group is bonded to the "$C_{1-18}$ hydrocarbylcarbonyl group".

The "$C_{1-18}$ hydrocarbylcarbonylamino group" is, for example, but not limited to, a "$C_{1-18}$ alkyl carbonylamino group", such as a methyl carbonylamino group, an ethyl carbonylamino group, a n-propyl carbonylamino group, an i-propyl carbonylamino group, a n-butyl carbonylamino group, an i-butyl carbonylamino group, a sec-butyl carbonylamino group, a t-butyl carbonylamino group, a n-pentyl carbonylamino group, an i-pentyl carbonylamino group, or a n-hexyl carbonylamino group; a "$C_{3-18}$ alicyclic carbonylamino group", such as a cyclopropyl carbonylamino group, a cyclobutyl carbonylamino group, a cyclopentyl carbonylamino group, or a cyclohexyl carbonylamino group; a "$C_{6-18}$ aryl carbonylamino group", such as a phenyl carbonylamino group, a naphthyl carbonylamino group, an acenaphthyl carbonylamino group, a phenanthrenyl carbonylamino group, or an anthracenyl carbonylamino group; or the like.

The term "$C_{1-18}$ hydrocarbylaminocarbonyloxy group", as used herein, refers to a group in which an oxygen atom (-O-) is bonded to the "$C_{1-18}$ hydrocarbylaminocarbonyl group".

The "$C_{1-18}$ hydrocarbylaminocarbonyloxy group" is, for example, but not limited to, a "$C_{1-18}$ alkylaminocarbonyloxy group", such as a methylaminocarbonyloxy group, an ethylaminocarbonyloxy group, or a n-propylaminocarbonyloxy group; a "$C_{3-18}$ alicyclic aminocarbonyloxy group", such as a cyclopropylaminocarbonyloxy group or a cyclohexylaminocarbonyloxy group; a "$C_{6-18}$ arylaminocarbonyloxy group", such as a phenylaminocarbonyloxy group or a 1-naphthylaminocarbonyloxy group; or the like.

The term "di-$C_{1-18}$ hydrocarbylaminocarbonyloxy group", as used herein, refers to a group in which an oxygen atom (-O-) is bonded to the "di-$C_{1-18}$ hydrocarbylaminocarbonyl group".

The "di-$C_{1-18}$ hydrocarbylaminocarbonyloxy group" is, for example, but not limited to, a "di-$C_{1-18}$ alkylaminocarbonyloxy group", such as a dimethylaminocarbonyloxy group, a diethylaminocarbonyloxy group, or a di-n-propylaminocarbonyloxy group; or the like.

The term "$C_{1-18}$ hydrocarbylaminocarbonylamino group", as used herein, refers to a group in which the "$C_{1-18}$

hydrocarbylaminocarbonyl group" is bonded to an amino group.

The "$C_{1-18}$ hydrocarbylaminocarbonylamino group" is, for example, but not limited to, a "$C_{1-18}$ alkylaminocarbonylamino group", such as a methylaminocarbonylamino group, an ethylaminocarbonyloxy group, or a n-propylaminocarbonylamino group; a "$C_{3-18}$ alicyclic aminocarbonylamino group", such as a cyclopropylaminocarbonylamino group or a cyclohexylaminocarbonylamino group; a "$C_{6-18}$ arylaminocarbonylamino group", such as a phenylaminocarbonylamino group or a 1-naphthylaminocarbonylamino group; or the like.

The term "di-$C_{1-18}$ hydrocarbylaminocarbonylamino group", as used herein, refers to a group in which a "di-$C_{1-18}$ hydrocarbylaminocarbonyl group" is bonded to an amino group.

The "di-$C_{1-18}$ hydrocarbylaminocarbonylamino group" is, for example, but not limited to, a "di-$C_{1-18}$ alkylaminocarbonylamino group", such as a dimethylaminocarbonylamino group, a diethylaminocarbonylamino group, or a di-n-propylaminocarbonylamino group; or the like.

The term "$C_{1-18}$ hydrocarbyloxycarbonylamino group", as used herein, refers to a group in which the "$C_{1-18}$ hydrocarbyloxycarbonyl group" is bonded to an amino group.

The "$C_{1-18}$ hydrocarbyloxycarbonylamino group" is, for example, but not limited to, a "$C_{1-18}$ alkoxycarbonylamino group", such as a methoxycarbonylamino group, an ethoxycarbonylamino group, a n-propoxycarbonylamino group, an i-propoxycarbonylamino group, a n-butoxycarbonylamino group, or a t-butoxycarbonylamino group; a "$C_{3-18}$ alicyclic oxycarbonylamino group", such as a cyclopropyloxycarbonylamino group or a cyclohexyloxycarbonylamino group; a "$C_{6-18}$ aryloxycarbonylamino group", such as a phenyloxycarbonylamino group or a 1-naphthyloxycarbonylamino group; or the like.

The term "$C_{1-18}$ hydrocarbylthio group", as used herein, refers to a group in which a sulfur atom (-S-) is bonded to the "$C_{1-18}$ hydrocarbyl group".

The "$C_{1-18}$ hydrocarbylthio group" is, for example, but not limited to, a "$C_{1-18}$ alkylthio group", such as a methylthio group, an ethylthio group, a n-propylthio group, an i-propylthio group, a n-butylthio group, an i-butylthio group, a t-butylthio group, a n-pentylthio group, or a n-hexylthio group; a "$C_{3-18}$ alicyclic thio group", such as a cyclopropylthio group, a cyclobutylthio group, a cyclopentylthio group, a cyclohexylthio group, a 2-methylcyclopentylthio group, a 3-methylcyclopentylthio group, a 2-methylcyclohexylthio group, a 3-methylcyclohexylthio group, or a 4-methylcyclohexylthio group; a "$C_{6-18}$ arylthio group", such as a phenylthio group, a 1-naphthylthio group, a 2-naphthylthio group, an acenaphthylthio group, a phenanthrenylthio group, or an anthracenylthio group; or the like.

The term "$C_{1-18}$ hydrocarbylsulfinyl group", as used herein, refers to a group in which a sulfinyl group (-S(=O)-) is bonded to the "$C_{1-18}$ hydrocarbyl group".

The "$C_{1-18}$ hydrocarbylsulfinyl group" is, for example, but not limited to, a "$C_{1-18}$ alkylsulfinyl group", such as a methylsulfinyl group, an ethylsulfinyl group, a n-propylsulfinyl group, an i-propylsulfinyl group, a n-butylsulfinyl group, a t-butylsulfinyl group, a pentylsulfinyl group, or a hexylsulfinyl group; a "$C_{3-18}$ alicyclic sulfinyl group", such as a cyclopropylsulfinyl group, a cyclobutylsulfinyl group, a cyclopentylsulfinyl group, a cyclohexylsulfinyl group, a 2-methylcyclopentylsulfinyl group, a 3-methylcyclopentylsulfinyl group, a 2-methylcyclohexylsulfinyl group, a 3-methylcyclohexylsulfinyl group, or a 4-methylcyclohexylsulfinyl group; a "$C_{6-18}$ arylsulfinyl group", such as a phenylsulfinyl group, a naphthylsulfinyl group, an acenaphthylsulfinyl group, a phenanthrenylsulfinyl group, or an anthracenylsulfinyl group; or the like.

The term "$C_{1-18}$ hydrocarbylsulfonyl group", as used herein, refers to a group in which a sulfonyl group ($-SO_2-$) is bonded to the "$C_{1-18}$ hydrocarbyl group".

The "$C_{1-18}$ hydrocarbylsulfonyl group" is, for example, but not limited to, a "$C_{1-18}$ alkylsulfonyl group", such as a methylsulfonyl group, an ethylsulfonyl group, a n-propylsulfonyl group, an i-propylsulfonyl group, a n-butylsulfonyl group, a t-butylsulfonyl group, or a pentylsulfonyl group; a "$C_{3-18}$ alicyclic sulfonyl group", such as a cyclopropylsulfonyl group, a cyclobutylsulfonyl group, a cyclopentylsulfonyl group, a cyclohexylsulfonyl group, a 2-methylcyclopentylsulfonyl group, a 3-methylcyclopentylsulfonyl group, a 2-methylcyclohexylsulfonyl group, a 3-methylcyclohexylsulfonyl group, or a 4-methylcyclohexyl group; a "$C_{6-18}$ arylsulfonyl group", such as a phenylsulfonyl group, a naphthylsulfonyl group, an acenaphthylsulfonyl group, a phenanthrenylsulfonyl group, or an anthracenylsulfonyl group; or the like.

The phrase "optionally substituted", as used herein, is not particularly limited as long as it is chemically acceptable and has the advantages of the present invention.

The "substituent" is, for example, (1) a halogen atom, (2) a hydroxy group, (3) a thiol group, (4) a nitro group, (5) a cyano group, (6) a carboxy group, (7) an amino group, (8) a sulfo group, or (9) a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least some of the hydrogen atoms may be substituted by (1) to (8), and a divalent carbon atom at any position excluding the terminals of the substituent may be replaced by -O-, -C(=O)-, COO-, -OCO-, -CONH-, - NHCO-, -S-,

or $SO_2$- (provided that adjacent divalent carbon atoms are not replaced at the same time).

[1-2-1. Sulfonium Cation]

[1-2-1-1. Sulfonium Cation (II)]

A sulfonium cation represented by the following general formula (II-1) or a sulfonium dication represented by the following general formula (II-2) can be used as the sulfonium cation according to the present embodiment.

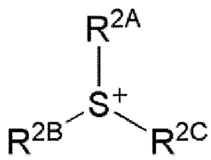

(II-1)

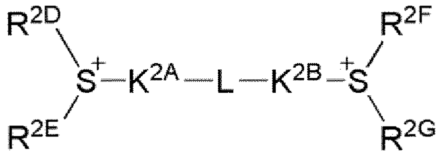

(II-2)

**[1-2-1-1-1.** Sulfonium Cation (II-1)]

In a sulfonium cation represented by the general formula (II-1),

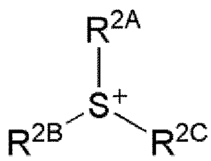

(II-1)

$R^{2A}$, $R^{2B}$, and $R^{2C}$ each independently denote an optionally substituted $C_{1-18}$ hydrocarbyl group, 3- to 18-membered non-aromatic heterocyclic group, or 5- to 18-membered aromatic heterocyclic group,
a divalent carbon atom at any position excluding the terminals of $R^{2A}$, $R^{2B}$, and $R^{2C}$ may be replaced by -O-, -C(=O)-, COO-, -OCO-, -CONH-, -NHCO-, -S-, or $SO_2$-(provided that adjacent divalent carbon atoms are not replaced at the same time); and
any two of $R^{2A}$, $R^{2B}$, and $R^{2C}$ may be linked to each other directly by a single bond or via a divalent linking group -O-, -S-, -C(=O)-, -S(=O)-, -S(=O)$_2$-, -C(=O)O-, or a $C_{1-3}$ alkylene group to form a ring together with the sulfur atom in the formula (II-1).

In a sulfonium cation represented by the general formula (II-1), for example, any one or more of $R^{2A}$ to $R^{2C}$ may be an optionally substituted $C_{1-18}$ hydrocarbyl group.
Examples of the case where all of $R^{2A}$ to $R^{2C}$ are an optionally substituted $C_{1-18}$ alkyl group, $C_{2-18}$ alkenyl group, $C_{2-18}$ alkynyl group, $C_{3-18}$ cycloalkyl group, $C_{3-18}$ cycloalkenyl group, or $C_{3-18}$ cycloalkenyl group include the following sulfonium cations and the like.
A divalent carbon atom at any position excluding the terminals of the $C_{1-18}$ alkyl group, the $C_{2-18}$ alkenyl group, the $C_{2-18}$ alkynyl group, the $C_{3-18}$ cycloalkyl group, the $C_{3-18}$ cycloalkenyl group, or the $C_{3-18}$ cycloalkenyl group can be replaced by -O-, -C(=O)-, COO-, -OCO-, -CONH-, -NHCO-, -S-, or $SO_2$- to adjust the solubility of the isopolyoxotungstate in a solvent or the like.

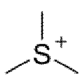

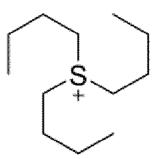

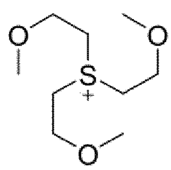

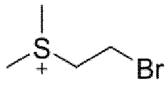 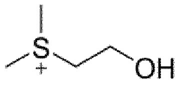 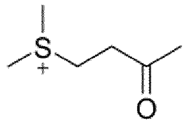 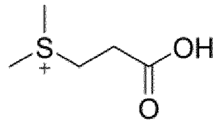

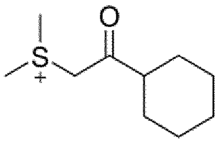 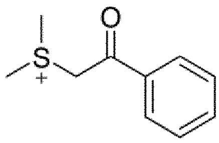 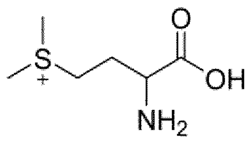 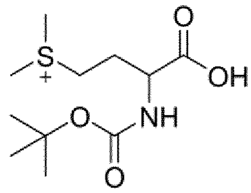

In the case where any two of $R^{2A}$ to $R^{2C}$ are optionally substituted $C_{1-18}$ alkyl groups, $C_{2-18}$ alkenyl groups, $C_{2-18}$ alkynyl groups, $C_{3-18}$ cycloalkyl groups, $C_{3-18}$ cycloalkenyl groups, or $C_{3-18}$ cycloalkenyl groups, they may be linked to each other directly by a single bond or via a divalent linking group -O-, -S-, -C(=O)-, -S(=O)-, - $S(=O)_2$-, -C(=O)O-, or a $C_{1-3}$ alkylene group to form a ring together with the sulfur atom in the formula (II-1).

Examples with the ring thus formed include the following sulfonium cations.

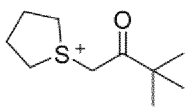 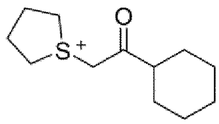 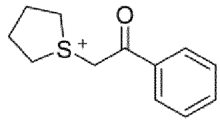 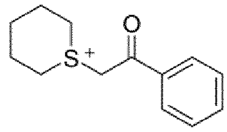

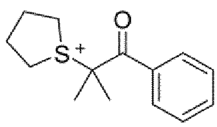 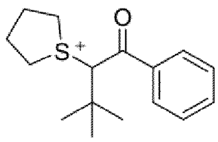 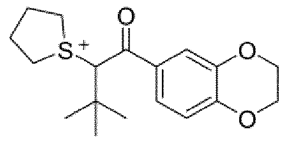

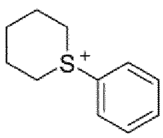 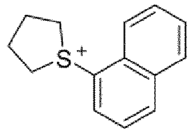 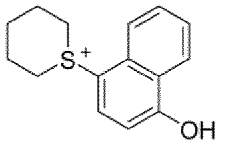 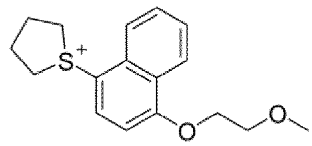

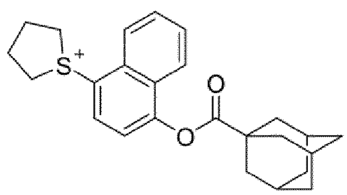 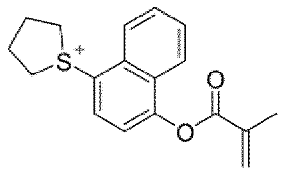

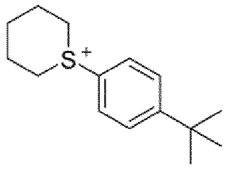 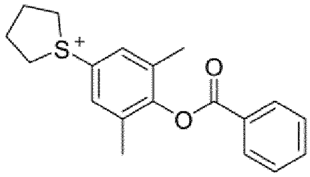 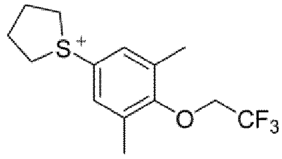

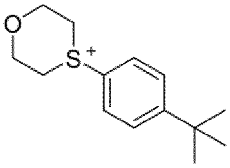 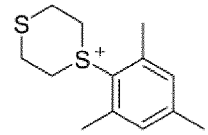 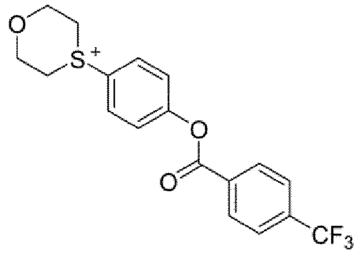

Examples of the case where any one or more of $R^{2A}$ to $R^{2C}$ is an optionally substituted $C_{6-18}$ aryl group include the following sulfonium cations.

An electron-donating group, an electron-withdrawing group, or the like can be introduced as a substituent of the $C_{6-18}$ aryl group to adjust the stability, reactivity, and the like of the isopolyoxotungstate. The substituent of the $C_{6-18}$ aryl group is preferably a halogen atom, a hydroxy group, a nitro group, a cyano group, or an optionally substituted $C_{1-18}$ haloalkyl group, $C_{1-18}$ hydrocarbyloxy group, $C_{1-18}$ hydrocarbyloxycarbonyl group, $C_{1-18}$ hydrocarbylcarbonyloxy group, di-$C_{1-18}$ hydrocarbylaminocarbonyloxy group, or $C_{1-18}$ hydrocarbylthio group, more preferably a halogen atom, a nitro group, a cyano group, or an optionally substituted $C_{1-4}$ haloalkyl group, $C_{1-12}$ hydrocarbyloxy group, $C_{1-12}$ hydrocarbyloxycarbonyl group, $C_{1-12}$ hydrocarbylcarbonyloxy group, di-$C_{1-12}$ hydrocarbylaminocarbonyloxy group, or $C_{1-12}$ hydrocarbylthio group, still more preferably a halogen atom (fluorine or iodine) or an optionally substituted $C_{1-4}$ haloalkyl group.

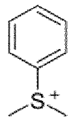 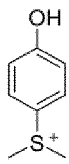 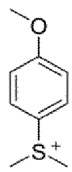 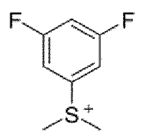 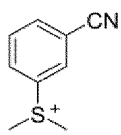 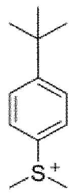

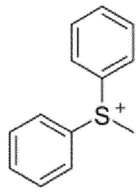 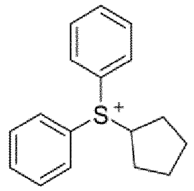 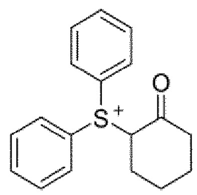 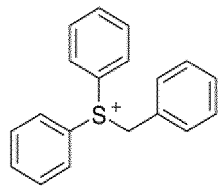

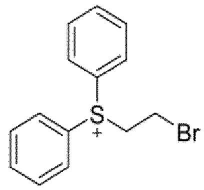 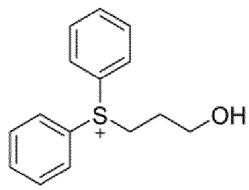 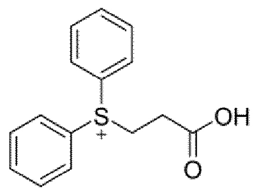 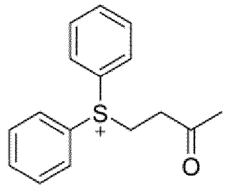

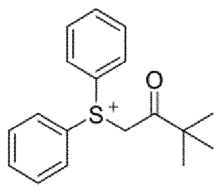 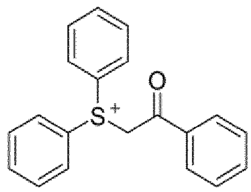 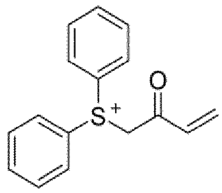

In the case where any two of $R^{2A}$ to $R^{2C}$ are optionally substituted $C_{6-18}$ aryl groups, they may be linked to each other directly by a single bond or via a divalent linking group -O-, -S-, -C(=O)-, -S(=O)-, $-S(=O)_2-$, -C(=O)O-, or a $C_{1-3}$ alkylene group to form a ring together with the sulfur atom in the formula (II-1).

In a sulfonium cation represented by the general formula (II-1), for example, any one or more of $R^{2A}$ to $R^{2C}$ may be an optionally substituted 3- to 18-membered non-aromatic heterocyclic group.

Examples of the case where any one or more of $R^{2A}$ to $R^{2C}$ is an optionally substituted 3- to 18-membered non-aromatic heterocyclic group include the following sulfonium cations.

A divalent carbon atom at any position excluding the terminals of the 3- to 18-membered non-aromatic

heterocyclic group can be replaced with -O-, -C(=O)-, COO-, - OCO-, -CONH-, -NHCO-, -S-, or $SO_2$- to adjust the solubility of the isopolyoxotungstate in a solvent or the like.

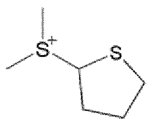
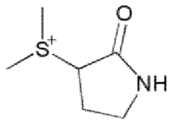
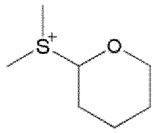
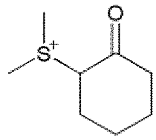

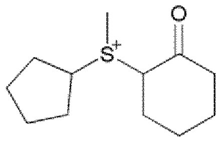
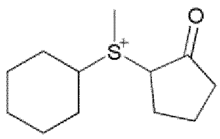
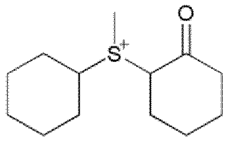
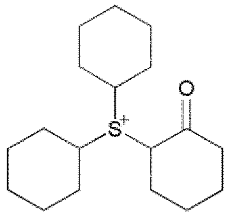

In a sulfonium cation represented by the general formula (II-1), for example, any one or more of $R^{2A}$ to $R^{2C}$ may be an optionally substituted 5- to 18-membered aromatic heterocyclic group.

[1-2-1-1-2. Sulfonium Dication (II-2)]

In a sulfonium dication represented by the general formula (II-2),

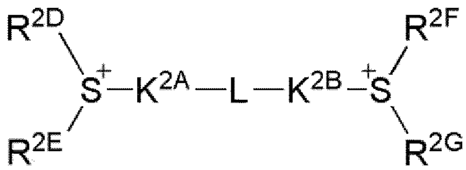
(I I - 2)

$R^{2D}$, $R^{2E}$, $R^{2F}$, and $R^{2G}$ each independently denote an optionally substituted $C_{1-18}$ hydrocarbyl group, 3- to 18-membered non-aromatic heterocyclic group, or 5- to 18-membered aromatic heterocyclic group,
a divalent carbon atom at any position excluding the terminals of $R^{2D}$, $R^{2E}$, $R^{2F}$, and $R^{2G}$ may be replaced by -O-, -C(=O)-, COO-, -OCO-, -CONH-, -NHCO-, -S-, or $SO_2$-(provided that adjacent divalent carbon atoms are not replaced at the same time);
$K^{2A}$, $K^{2B}$, and L each independently denote an optionally substituted $C_{1-18}$ hydrocarbylene group,
a divalent carbon atom at any position of $K^{2A}$, $K^{2B}$, and L may be replaced by - O-, -C(=O)-, COO-, -OCO-, -CONH-, -NHCO-, -S-, or $SO_2$- (provided that adjacent divalent carbon atoms are not replaced at the same time); and
any two of $R^{2D}$, $R^{2E}$, and $K^{2A}$ and/or any two of $R^{2F}$, $R^{2G}$, and $K^{2B}$ may be linked to each other directly by a single bond or via a divalent linking group -O-, -S-, -C(=O)-, - S(=O)-, $-S(=O)_2-$, -C(=O)O-, or a $C_{1-3}$ alkylene group to form a ring together with a sulfur atom in the formula (II-2).

In a sulfonium dication represented by the general formula (II-2), for example, any one or more of $K^{2A}$, $K^{2B}$, and L may be an optionally substituted $C_{1-18}$ alkylene group or $C_{6-18}$ arylene group among optionally substituted $C_{1-18}$ hydrocarbylene groups.

Examples of the case where any one or more of $K^{2A}$, $K^{2B}$, and L is an optionally substituted $C_{1-18}$ alkylene group or $C_{6-18}$ arylene group include the following sulfonium dications.

A divalent carbon atom at any position of the $C_{1-18}$ alkylene group or the $C_{6-18}$ arylene group can be replaced by -O-, -C(=O)-, COO-, -OCO-, -CONH-, -NHCO-, -S-, or $SO_2$- to adjust the solubility of the isopolyoxotungstate in a solvent or the like.

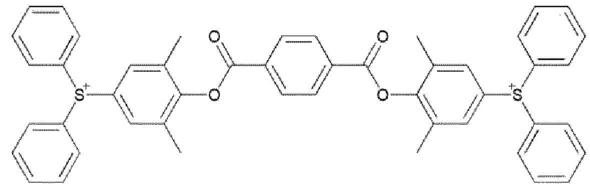

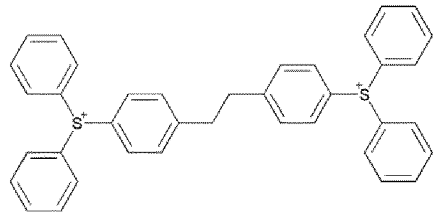

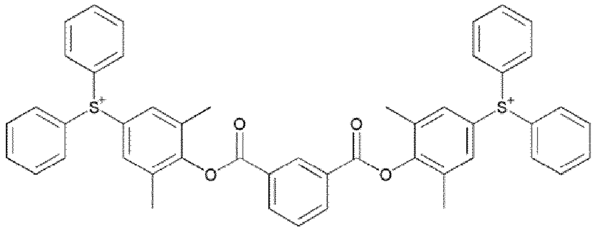

In a sulfonium cation represented by the general formula (II-2), any two of $R^{2D}$, $R^{2E}$, and $K^{2A}$ and/or any two of $R^{2F}$, $R^{2G}$, and $K^{2B}$ may be linked to each other directly by a single bond or via a divalent linking group -O-, -S-, -C(=O)-, -S(=O)-, $-S(=O)_2-$, - C(=O)O-, or a $C_{1-3}$ alkylene group to form a ring together with a sulfur atom in the formula (II-2).

[1-2-1-2. Sulfonium Cation (III)]

Sulfonium cations represented by the following general formulae (III-1) to (III-5) may be used as other sulfonium cations according to the present embodiment.

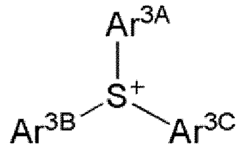

(I I I－1)

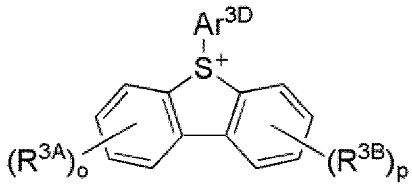

(I I I－2)

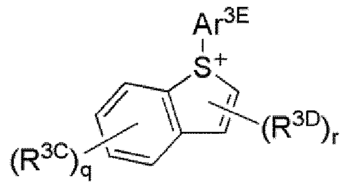

(I I I－3)

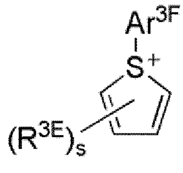

(I I I－4)

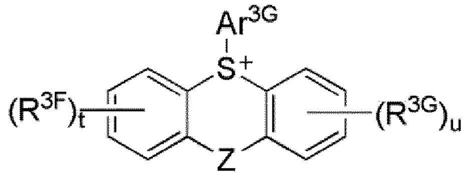

(III-5)

[1-2-1-2-1. Sulfonium Cation (III-1)]

In a sulfonium cation represented by the general formula (III-1),

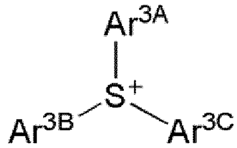

(III-1)

$Ar^{3A}$ to $Ar^{3C}$ each independently denote an optionally substituted $C_{6-18}$ aryl group or 5- to 18-membered heterocyclic group,

in the optionally substituted $C_{6-18}$ aryl group or 5- to 18-membered heterocyclic group, at least part of the hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group, and

a divalent carbon atom at any position excluding the terminals of the substituent may be replaced by -O-, -C(=O)-, COO-, -OCO-, -CONH-, -NHCO-, -S-, or $SO_2$-(provided that adjacent divalent carbon atoms are not replaced at the same time).

In a sulfonium cation represented by the general formula (III-1), for example, any one or more of $Ar^{3A}$ to $Ar^{3C}$ may be an optionally substituted $C_{6-18}$ aryl group or 5- to 18-membered heterocyclic group.

In a sulfonium cation represented by the general formula (III-1), examples of the case where all of $Ar^{3A}$ to $Ar^{3C}$ are optionally substituted $C_{6-18}$ aryl groups include the following sulfonium cations and the like.

An electron-donating group, an electron-withdrawing group, or the like can be introduced as a substituent of the $C_{6-18}$ aryl group to adjust the stability, reactivity, and the like of the isopolyoxotungstate. The substituent of the $C_{6-18}$ aryl group is preferably a halogen atom, a hydroxy group, a nitro group, a cyano group, or an optionally substituted $C_{1-18}$ haloalkyl group, $C_{1-18}$ hydrocarbyloxy group, $C_{1-18}$ hydrocarbyloxycarbonyl group, $C_{1-18}$ hydrocarbylcarbonyloxy group, di-$C_{1-12}$ hydrocarbylaminocarbonyloxy group, or $C_{1-18}$ hydrocarbylthio group, more preferably a halogen atom, a nitro group, a cyano group, or an optionally substituted $C_{1-4}$ haloalkyl group, $C_{1-12}$ hydrocarbyloxy group, $C_{1-12}$ hydrocarbyloxycarbonyl group, $C_{1-12}$ hydrocarbylcarbonyloxy group, di-$C_{1-12}$ hydrocarbylaminocarbonyloxy group, or $C_{1-12}$ hydrocarbylthio group, still more preferably a halogen atom (fluorine or iodine) or an optionally substituted $C_{1-4}$ haloalkyl group.

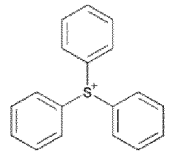 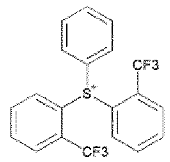 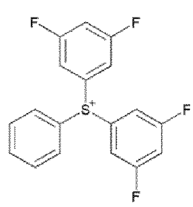 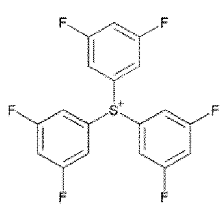 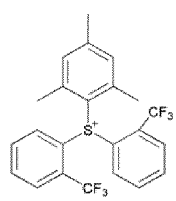

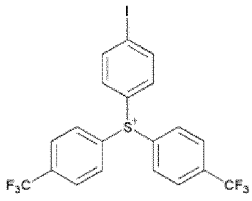
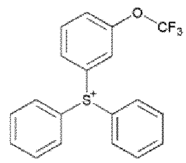
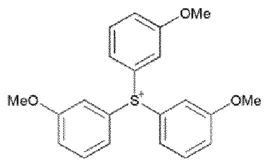
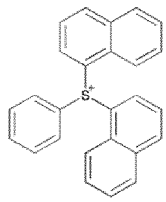
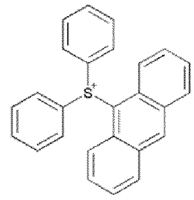

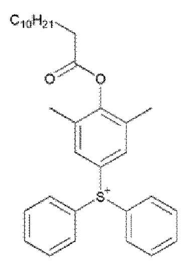
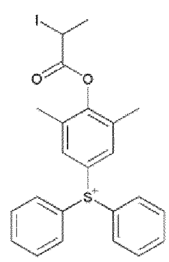
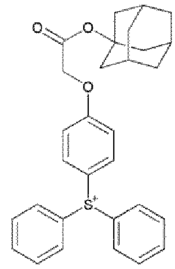
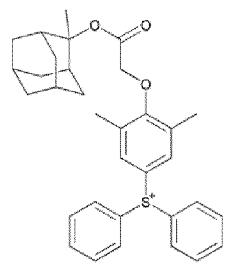
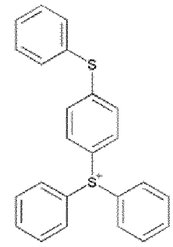

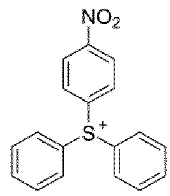
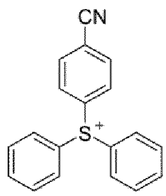
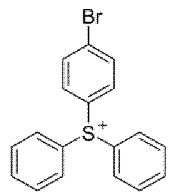
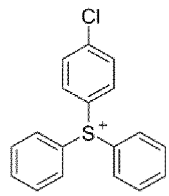
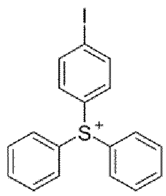

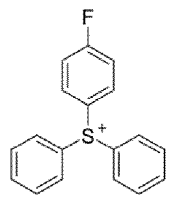
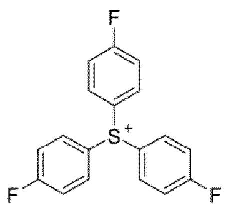
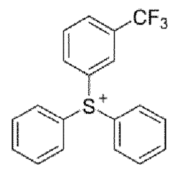
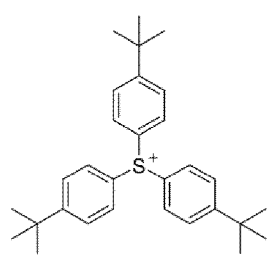
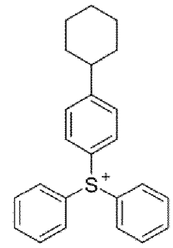

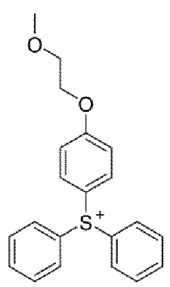
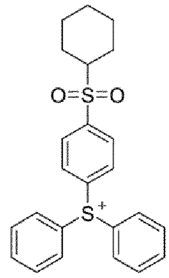
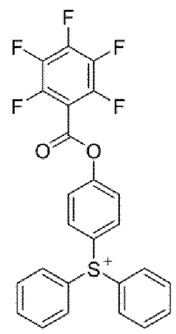
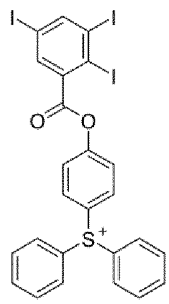

In a sulfonium cation represented by the general formula (III-1), for example, any one or more of $Ar^{3A}$ to $Ar^{3C}$ may be an optionally substituted 5- to 18-membered heterocyclic group.

Examples of the case where any one or more of $Ar^{3A}$ to $Ar^{3C}$ is an optionally substituted 5- to 18-membered heterocyclic group include the following sulfonium cations.

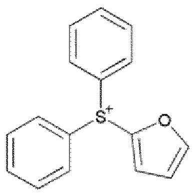
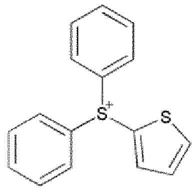
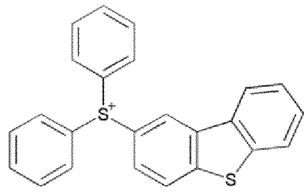

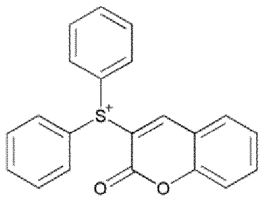
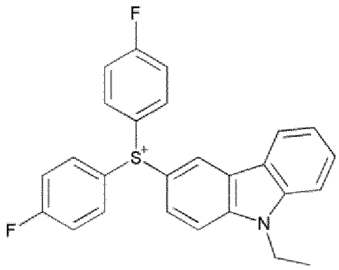

[1-2-1-2-2. Sulfonium Cation (III-2)]

In a sulfonium cation represented by the general formula (III-2),

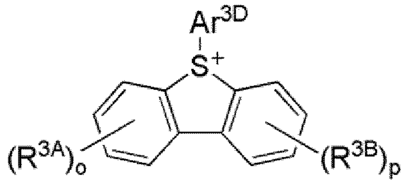

(III-2)

$Ar^{3D}$ denotes an optionally substituted $C_{6-18}$ aryl group or 5- to 18-membered heterocyclic group,
in the optionally substituted $C_{6-18}$ aryl group or 5- to 18-membered heterocyclic group, at least part of the hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,
a divalent carbon atom at any position excluding the terminals of the substituent may be replaced by -O-, -C(=O)-, COO-, -OCO-, -CONH-, -NHCO-, -S-, or $SO_2$-(provided that adjacent divalent carbon atoms are not replaced at the same time);
$R^{3A}$ and $R^{3B}$ each independently denote a halogen atom, a hydroxy group, a thiol group, a nitro group, a cyano group, a carboxy group, an amino group, or a sulfo group, or each independently denote a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,
a divalent carbon atom at any position excluding the terminals may be replaced by -O-, -C(=O)-, COO-, -OCO-, -CONH-, -NHCO-, -S-, or $SO_2$- (provided that adjacent divalent carbon atoms are not replaced at the same time); and

o and p each denote an integer in the range of 0 to 4, and when o and p denote an integer of 2 or more, $R^{3A}$ and $R^{3B}$ may be the same or different.

In a sulfonium cation represented by the general formula (III-2), for example, $Ar^{3D}$ may denote an optionally substituted $C_{6-18}$ aryl group, and $R^{3A}$ and $R^{3B}$ may denote, as an electron-donating group or an electron-withdrawing group, a halogen atom, a hydroxy group, a nitro group, a cyano group, an optionally substituted $C_{1-4}$ haloalkyl group, an optionally substituted $C_{1-18}$ hydrocarbyloxy group, an optionally substituted $C_{1-18}$ hydrocarbyloxycarbonyl group, an optionally substituted $C_{1-18}$ hydrocarbylcarbonyloxy group, an optionally substituted $C_{1-18}$ hydrocarbylthio group, or the like.

The sulfonium cation is, for example, one of the following sulfonium cations.

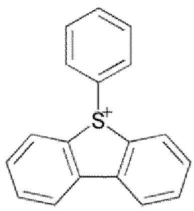
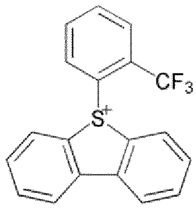
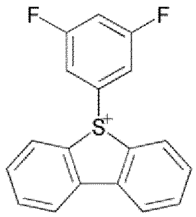
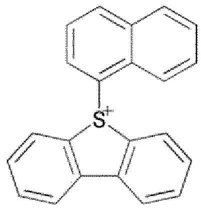

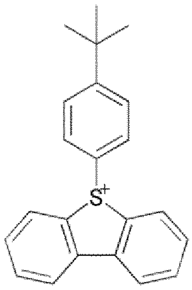
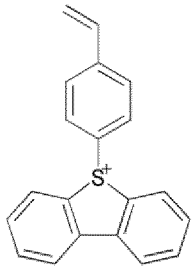
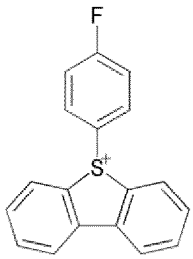
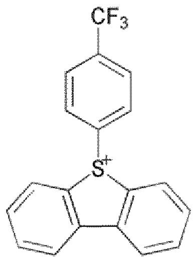

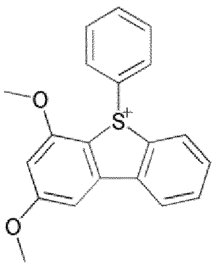
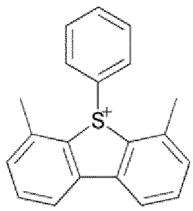
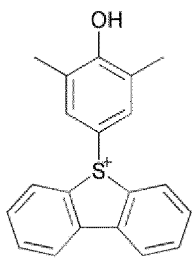
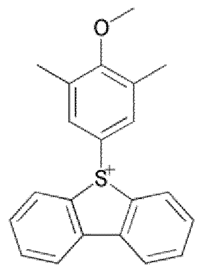

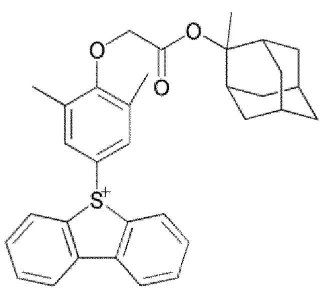
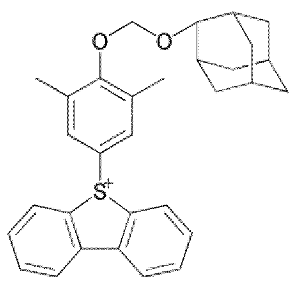
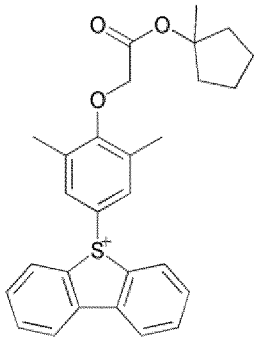

[1-2-1-2-3. Sulfonium Cation (III-3)]

In a sulfonium cation represented by the general formula (III-3),

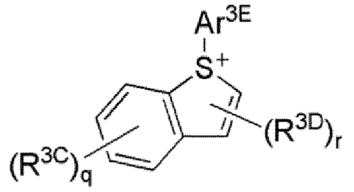

(III-3)

$Ar^{3E}$ denotes an optionally substituted $C_{6-18}$ aryl group or 5- to 18-membered heterocyclic group,
in the optionally substituted $C_{6-18}$ aryl group or 5- to 18-membered heterocyclic group, at least part of the hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,
a divalent carbon atom at any position excluding the terminals of the substituent may be replaced by -O-, -C(=O)-, COO-, -OCO-, -CONH-, -NHCO-, -S-, or $SO_2$-(provided that adjacent divalent carbon atoms are not replaced at the same time);
$R^{3C}$ and $R^{3D}$ each independently denote a halogen atom, a hydroxy group, a thiol group, a nitro group, a cyano group, a carboxy group, an amino group, or a sulfo group, or each independently denote a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,
a divalent carbon atom at any position excluding the terminals may be replaced by -O-, -C(=O)-, COO-, -OCO-, -CONH-, -NHCO-, -S-, or $SO_2$- (provided that adjacent divalent carbon atoms are not replaced at the same time); and
q denotes an integer in the range of 0 to 4, r denotes an integer in the range of 0 to 2, and when q and r denote an integer of 2 or more, $R^{3C}$ and $R^{3D}$ may be the same or different.

In a sulfonium cation represented by the general formula (III-3), for example, $Ar^{3E}$ may denote an optionally substituted $C_{6-18}$ aryl group, and $R^{3C}$ and $R^{3D}$ may denote, as an electron-donating group or an electron-withdrawing group, a halogen atom, a hydroxy group, a nitro group, a cyano group, an optionally substituted $C_{1-4}$ haloalkyl group, an optionally substituted $C_{1-18}$ hydrocarbyloxy group, an optionally substituted $C_{1-18}$ hydrocarbyloxycarbonyl group, an optionally substituted $C_{1-18}$ hydrocarbylcarbonyloxy group, or an optionally substituted $C_{1-18}$ hydrocarbylthio group.

The sulfonium cation is, for example, one of the following sulfonium cations.

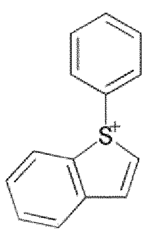

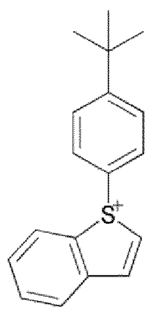

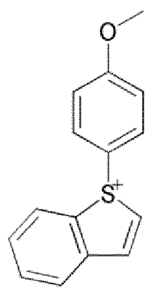

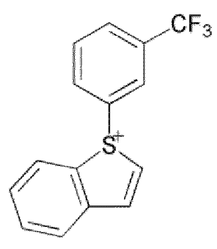

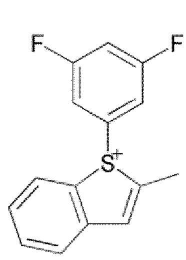
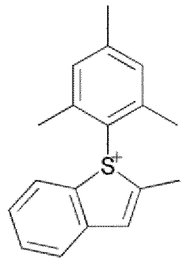

[1-2-1-2-4. Sulfonium Cation (III-4)]

In a sulfonium cation represented by the general formula (III-4),

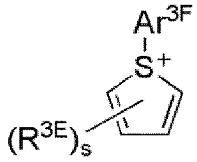
(I I I −4)

$Ar^{3F}$ denotes an optionally substituted $C_{6-18}$ aryl group or 5- to 18-membered heterocyclic group,
in the optionally substituted $C_{6-18}$ aryl group or 5- to 18-membered heterocyclic group, at least part of the hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,
a divalent carbon atom at any position excluding the terminals of the substituent may be replaced by -O-, -C(=O)-, COO-, -OCO-, -CONH-, -NHCO-, -S-, or $SO_2$-(provided that adjacent divalent carbon atoms are not replaced at the same time);
$R^{3E}$ denotes a halogen atom, a hydroxy group, a thiol group, a nitro group, a cyano group, a carboxy group, an amino group, or a sulfo group, or a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,
a divalent carbon atom at any position excluding the terminals may be replaced by -O-, -C(=O)-, COO-, -OCO-, -CONH-, -NHCO-, -S-, or $SO_2$- (provided that adjacent divalent carbon atoms are not replaced at the same time); and
s each denotes an integer in the range of 0 to 4, and when s denotes an integer of 2 or more, $R^{3E}$ may be the same or different.

In a sulfonium cation represented by the general formula (III-4), for example, $Ar^{3F}$ may denote an optionally substituted $C_{6-18}$ aryl group, and $R^{3E}$ may denote, as an electron-donating group or an electron-withdrawing group, a halogen atom, a hydroxy group, a nitro group, a cyano group, an optionally substituted $C_{1-4}$ haloalkyl group, an optionally substituted $C_{1-18}$ hydrocarbyloxy group, an optionally substituted $C_{1-18}$ hydrocarbyloxycarbonyl group, an optionally substituted $C_{1-18}$ hydrocarbylcarbonyloxy group, or an optionally substituted $C_{1-18}$ hydrocarbylthio group.

[1-2-1-2-5. Sulfonium Cation (III-5)]

In a sulfonium cation represented by the general formula (III-5),

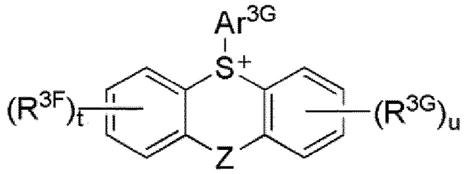

(III-5)

$Ar^{3G}$ each independently denotes an optionally substituted $C_{6-18}$ aryl group or 5-to 18-membered heterocyclic group, in the optionally substituted $C_{6-18}$ aryl group or 5- to 18-membered heterocyclic group, at least part of the hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,

a divalent carbon atom at any position excluding the terminals of the substituent may be replaced by -O-, -C(=O)-, COO-, -OCO-, -CONH-, -NHCO-, -S-, or $SO_2$-(provided that adjacent divalent carbon atoms are not replaced at the same time);

$R^{3F}$ and $R^{3G}$ each independently denote a halogen atom, a hydroxy group, a thiol group, a nitro group, a cyano group, a carboxy group, an amino group, or a sulfo group, or each independently denote a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group, a divalent carbon atom at any position excluding the terminals may be replaced by -O-, -C(=O)-, COO-, -OCO-, -CONH-, -NHCO-, -S-, or $SO_2$- (provided that adjacent divalent carbon atoms are not replaced at the same time);

t and u each denote an integer in the range of 0 to 4, and when t and u denote an integer of 2 or more, $R^{3F}$ and $R^{3G}$ may be the same or different; and

Z denotes -O-, -S-, -C(=O)-, -S(=O)-, $-S(=O)_2-$, -C(=O)O-, or a $C_{1-3}$ alkylene group.

In a sulfonium cation represented by the general formula (III-5), for example, $Ar^{3G}$ may denote an optionally substituted $C_{6-18}$ aryl group, and $R^{3F}$ and $R^{3G}$ may denote, as an electron-donating group or an electron-withdrawing group, a halogen atom, a hydroxy group, a nitro group, a cyano group, an optionally substituted $C_{1-4}$ haloalkyl group, an optionally substituted $C_{1-18}$ hydrocarbyloxy group, an optionally substituted $C_{1-18}$ hydrocarbyloxycarbonyl group, an optionally substituted $C_{1-18}$ hydrocarbylcarbonyloxy group, or an optionally substituted $C_{1-18}$ hydrocarbylthio group.

The sulfonium cation is, for example, one of the following sulfonium cations.

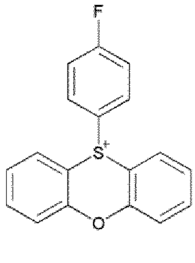 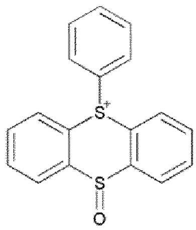 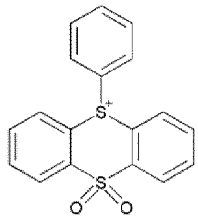 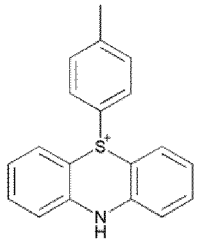 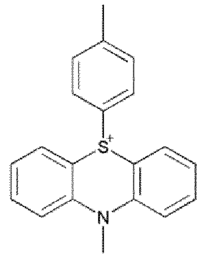

[1-2-1-3. Specific Examples of Sulfonium Cation]

Specific examples of the sulfonium cations or the sulfonium dications represented by the general formulae (II-1) to (III-5) include sulfonium cations and sulfonium dications, such as a dibutyl(pentyl)sulfonium cation, a 1-phenylhexahydrothiopyrylium cation, a (ethane-1,2-diylbisoxy)bis(4,1-phenylene)bis(diphenylsulfonium) dication, a (thiodi-4,1-phenylene)bis(diphenylsulfonium) dication, a diphenyl(thiophene-2-yl)sulfonium cation, a diphenyl(thiophene-3-yl)sulfonium cation, a di(naphthalene-1-yl)(phenyl)sulfonium cation, a phenyl bis(2-(trifluoromethyl)phenyl)sulfonium cation, a mesyl bis(2-(trifluoromethyl)phenyl)sulfonium cation, a bis(3,5-difluorophenyl)(phenyl)sulfonium cation, a tris(3,5-difluorophenyl)sulfonium cation, a (4-(dodecanoyloxy-3,5-dimethylphenyl))diphenylsulfonium cation, a diphenyl(3-(trifluoromethoxy)phenyl)sulfonium cation, a (4-((2-iodopropanoyl)oxy)-3,5-dimethylphenyl)diphenylsulfonium cation, a (4-iodophenyl)bis(4-(trifluoromethyl)phenyl)sulfonium cation, a 5-phenyl-5H-thianthren-5-ium cation, a 5-(2,5-dimethylphenyl) thianthren-5-ium cation, a 5-(3-(trifluoromethyl)phenyl)-5H-dibenzo[b,d]thiophen-5-ium cation, a 1-(4-(t-butyl)phenyl)-1H-benzo[b]thiophen-1-ium cation, and a 1-(4-(t-butyl)phenyl)-2,5-dimethyl-1H-thiophen-1-ium cation, a triethylsulfonium cation, a (2-carboxyethyl)dimethylsulfonium cation, a (3-chloropropyl)diphenylsulfonium cation, a benzyl(4-hydroxyphenyl)methylsulfonium cation, a (4-hydroxyphenyl)methyl(2-methylbenzyl)sulfonium cation, a 4-hydroxyphenyldimethylsulfonium cation, a diphenyl(methyl)sulfonium cation, a trimethylsulfonium cation, a dimethylphenacylsulfonium cation, a (difluoromethyl)bis(2,5-dimethylphenyl)sulfonium cation, a diphenyl(4-(phenylthio)phenyl)sulfonium cation, a (2-bromoethyl)diphenylsulfonium cation, a dimesityl(trifluoromethyl)sulfonium cation, and a tri-p-tolylsulfonium cation.

[1-2-2. Iodonium Cation]

[1-2-2-1. Iodonium Cation (IV)]

An iodonium cation according to the present embodiment can be an iodonium cation represented by the following general formula (IV).

$$R^{4A} - I^{+} - R^{4B} \qquad (IV)$$

In an iodonium cation represented by the general formula (IV),

$R^{4A}$ and $R^{4B}$ each independently denote an optionally substituted $C_{1-18}$ hydrocarbyl group, an optionally substituted 3- to 18-membered non-aromatic heterocyclic group, or an optionally substituted 5- to 18-membered aromatic heterocyclic group, and
$R^{4A}$ and $R^{4B}$ may be linked to each other directly by a single bond or via a divalent linking group -O-, -S-, -C(=O)-, -S(=O)-, $-S(=O)_2-$, -C(=O)O-, or a $C_{1-3}$ alkylene group to form a ring together with the iodine atom in the formula (V-1).

In an iodonium cation represented by the general formula (IV), for example, any one of $R^{4A}$ and $R^{4B}$ may be an optionally substituted $C_{2-18}$ alkynyl group among optionally substituted $C_{1-18}$ hydrocarbyl groups.

Examples of the case where any one of $R^{4A}$ and $R^{4B}$ denotes an optionally substituted $C_{1-18}$ alkynyl group include the following iodonium cation.

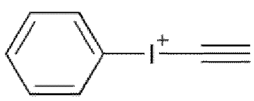

When $R^{4A}$ and $R^{4B}$ denote optionally substituted $C_{6-18}$ aryl groups, they may be linked to each other directly by a

single bond or via a divalent linking group -O-, -S-, - C(=O)-, -S(=O)-, $-S(=O)_2-$, -C(=O)O-, or a $C_{1-3}$ alkylene group to form a ring together with the iodine atom in the formula (IV).

Examples of the case where a ring is formed include the following iodonium cations and the like.

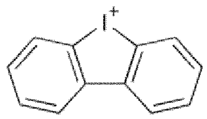 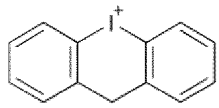 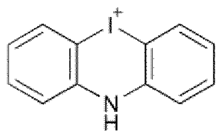 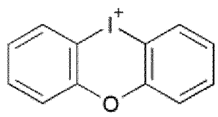

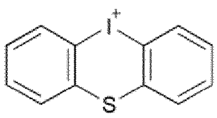 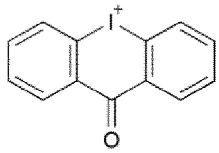 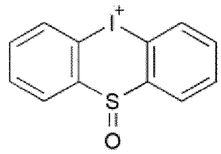 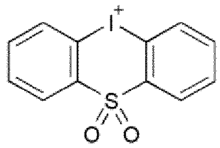

[1-2-2-2. Iodonium Cation (V)]

The iodonium cation according to the present embodiment may be an iodonium cation represented by (V-1) or (V-2).

$$Ar^{5A}-I^{+}-Ar^{5B} \quad (V-1)$$

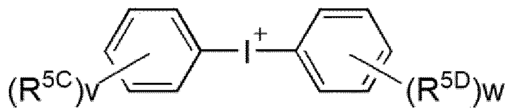 (V－2)

[1-2-2-2-1. Iodonium Cation (V-1)]

In an iodonium cation represented by the general formula (V-1),

$$Ar^{5A}-I^{+}-Ar^{5B} \quad (V-1)$$

$Ar^{5A}$ and $Ar^{5B}$ each independently denote an optionally substituted $C_{6-18}$ aryl group or 5- to 18-membered heterocyclic group,

in the optionally substituted $C_{6-18}$ aryl group or 5- to 18-membered heterocyclic group, at least part of the hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,

a divalent carbon atom at any position excluding the terminals of the substituent may be replaced by -O-, -C(=O)-, COO-, -OCO-, -CONH-, -NHCO-, -S-, or $SO_2$-(provided that adjacent divalent carbon atoms are not replaced at the same time), and

$Ar^{5A}$ and $Ar^{5B}$ may be linked to each other directly by a single bond or via a divalent linking group -O-, -S-, -C(=O)-, -S(=O)-, $-S(=O)_2-$, -C(=O)O-, or a $C_{1-3}$ alkylene group to form a ring together with the iodine atom in the formula (V-1).

In an iodonium cation represented by the general formula (V-1), for example, any one or more of $Ar^{5A}$ and $Ar^{5B}$

may be an optionally substituted 5- to 18-membered heterocyclic group.

The iodonium cation is, for example, one of the following iodonium cations.

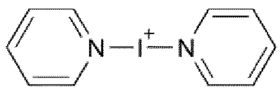
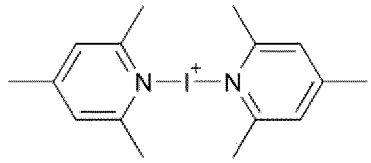
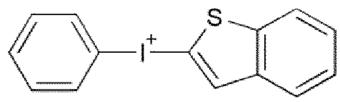

[1-2-2-2-2. Iodonium Cation (V-2)]

In an iodonium cation represented by the general formula (V-2),

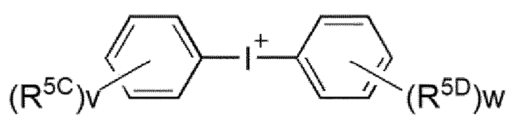
(V-2)

$R^{5C}$ and $R^{5D}$ each independently denote a halogen atom, a hydroxy group, a thiol group, a nitro group, a cyano group, a carboxy group, an amino group, or a sulfo group, or each independently denote a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of the hydrogen atoms may be substituted by a halogen atom, a hydroxy group, a thiol group, a nitro group, a cyano group, a carboxy group, an amino group, or a sulfo group, a divalent carbon atom at any position excluding the terminals may be replaced by -O-, -C(=O)-, COO-, -OCO-, -CONH-, -NHCO-, -S-, or $SO_2$- (provided that adjacent divalent carbon atoms are not replaced at the same time); and v and w each denote an integer in the range of 0 to 5, and when v and w denote an integer of 2 or more, $R^{5C}$ and $R^{5D}$ may be the same or different.

In an iodonium cation represented by the general formula (V-2), for example, $R^{5C}$ and $R^{5D}$ may denote, as an electron-donating group or an electron-withdrawing group, a halogen atom, a hydroxy group, a nitro group, a cyano group, an optionally substituted $C_{1-4}$ haloalkyl group, an optionally substituted $C_{1-18}$ hydrocarbyloxy group, an optionally substituted $C_{1-18}$ hydrocarbyloxycarbonyl group, an optionally substituted $C_{1-18}$ hydrocarbylcarbonyloxy group, or an optionally substituted $C_{1-18}$ hydrocarbylthio group.

The iodonium cation is, for example, one of the following iodonium cations.

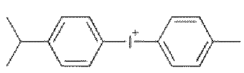
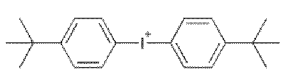
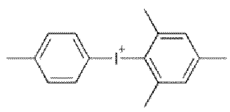
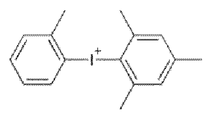

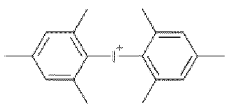
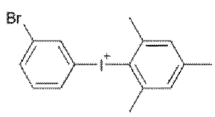
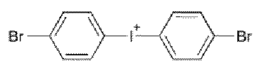
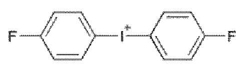

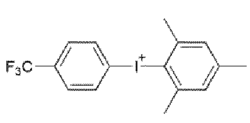
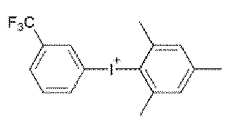
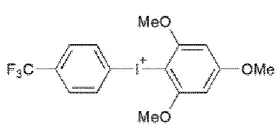
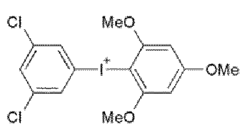

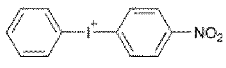
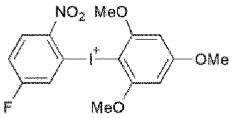
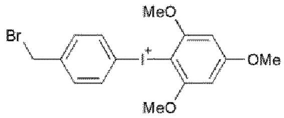
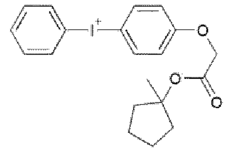
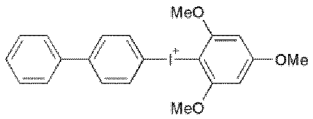
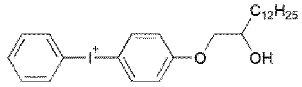

[1-2-2-3. Specific Examples of Iodonium Cation]

Specific examples of the iodonium cations represented by the general formulae (IV) to (V-2) include an ethynyl(phenyl)iodonium cation, a bis(pyridine)iodonium cation, a bis(2,4,6-trimethylpyridine)iodonium cation, a diphenyliodonium cation, a bis(4-(t-butyl)phenyl)iodonium cation, a (2-carboxyphenyl)(phenyl)iodonium cation, a (4-nitrophenyl)(phenyl)iodonium cation, a (3-(trifluoromethyl)phenyl)(2,4,6-trimethylphenyl)iodonium cation, a bis(4-fluorophenyl)iodonium cation, a (4-(bromomethyl)phenyl)(2,4,6-trimethoxyphenyl)iodonium cation, a 4-biphenylyl(2,4,6-trimethoxyphenyl)iodonium cation, a bis(2,4,6-trimethylphenyl)iodonium cation, a 4-isopropyl-4'-methyldiphenyliodonium cation, a (4-(trifluoromethyl)phenyl)(2,4,6-trimethylphenyl)iodonium cation, a ((4-trifluoromethyl)phenyl)(2,4,6-trimethoxyphenyl)iodonium cation, a (5-fluoro-2-nitrophenyl)(2,4,6-trimethoxyphenyl)iodonium cation, a (3-bromophenyl)(mesityl)iodonium cation, a bis(4-bromophenyl)iodonium cation, a (3,5-dichlorophenyl)(2,4,6-trimethoxyphenyl)iodonium cation, a (4-methylphenyl)(2,4,6-trimethylphenyl)iodonium cation, a (3-methylphenyl)(2,4,6-trimethylphenyl)iodonium cation, a (2-methylphenyl)(2,4,6-trimethylphenyl)iodonium cation, a phenyl(2,4,6-trimethoxyphenyl)iodonium cation, and the like.

[1-3. Other Counter Cations]

An isopolyoxotungstate compound according to the present embodiment is represented by the following general formula (I):

$$(A^{m+})_{x/m}(B^{n+})_{y/n}(C^{-(x+y)}) \qquad (I)$$

$A^{m+}$ each independently denotes $H^+$, a metal ion, or an ammonium ion; and
m denotes an integer in the range of 1 to 5, and x and x/m denote an integer.

A metal ion that can be used as $A^{m+}$ is, for example, but not limited to, $Li^+$, $Na^+$, $K^+$, $Rb^+$, $Cs^+$, $Be^{2+}$, $Mg^{2+}$, $Ca^{2+}$, $Sr^{2+}$, $Ba^{2+}$, $Co^{3+}$, $Bi^{3+}$, $Zr^{4+}$, $Hf^{4+}$, $Bi^{5+}$, or the like. These metal ions may be used alone or in combination.

An ammonium cation that can be used as $A^{m+}$ is, for example, but not limited to, $NH^{4+}$, a quaternary ammonium cation, or the like.

The quaternary ammonium cation can be represented by $(R)_4N^+$. Each R is independent and may be the same or different. R is, for example, but not limited to, an optionally substituted $C_{1-18}$ hydrocarbyl group or the like. R preferably denotes an optionally substituted $C_{1-12}$ hydrocarbyl group, more preferably an optionally substituted $C_{1-8}$ hydrocarbyl group.

From the perspective of the stability and reactivity of the isopolyoxotungstate, $A^{m+}$ is preferably $Li^+$, $Na^+$, $K^+$, $Rb^+$, $Cs^+$, $Mg^{2+}$, $Ca^{2+}$, $Sr^{2+}$, $Ba^{2+}$, $Co^{3+}$, $NH^{4+}$, or a quaternary ammonium cation, more preferably $Na^+$, $K^+$, $Rb^+$, $Cs^+$, $Co^{3+}$, $NH^{4+}$, or a quaternary ammonium cation, still more preferably $NH^{4+}$ or a quaternary ammonium cation.

In an isopolyoxotungstate compound or a compound represented by the following formula (I), the ratio of x/m to y/n is not particularly limited and can be appropriately determined depending on the stability, reactivity, use, and the like of the isopolyoxotungstate compound.

$$(A^{m+})_{x/m}(B^{n+})_{y/n}(C^{-(x+y)}) \qquad (I)$$

From the perspective of achieving both the reactivity and the stability of the isopolyoxotungstate compound, the ratio of x/m to y/n is preferably x/m:y/n = 0 to 6:1 to 12, more preferably 0 to 3:1 to 10, still more preferably 0 to 1:1 to 8.

In particular, when x + y is an integer in the range of 5 to 7 and m and n are 1, it is preferable that x is an integer in the range of 0 to 6 and y is an integer in the range of 1 to 7, it is more preferable that x is an integer in the range of 0 to 4 and y is an integer in the range of 2 to 7, and it is still more preferable that x is an integer in the range of 0 to 2 and y is an integer in the range of 3 to 7.

[1-4. Compound or Solvate or Mixture Thereof]

An isopolyoxotungstate mixture according to the present embodiment may contain two or more different isopolyoxotungstate compounds represented by the following general formula (I) or solvates thereof.

$$(A^{m+})_{x/m}(B^{n+})_{y/n}(C^{-(x+y)}) \qquad (I)$$

$A^{m+}$ each independently denotes $H^+$, a metal ion, or an ammonium ion;
$B^{n+}$ each independently denotes a sulfonium cation or an iodonium cation;
$C^{-(x+y)}$ denotes an isopolyoxotungstic acid; and
m denotes an integer in the range of 1 to 5, n denotes an integer of 1 or 2, x and x/m are integers of 0 or more, and y and y/n are natural numbers.

In an isopolyoxotungstate mixture, the isopolyoxotungstic acid represented by $C^{-(x+y)}$ can be, for example, but is not limited to, $[W_4O_{13}]^{2-}$, $[W_5O_{16}]^{2-}$, $[W_6O_{19}]^{2-}$, $[W_7O_{22}]^{2-}$, $[W_7O_{24}]^{6-}$, $[W_{10}O_{32}]^{4-}$, $[H_zW_{12}O_{40}]^{-(8-z)}$ (wherein z denotes an integer in the range of 1 to 4), $[H_4W_{11}O_{38}]^{6-}$, $[H_7W_{11}O_{40}]^{7-}$, $[HW_5O_{19}]^{7-}$, $[H_3W_{11}O_{22}]^{5-}$, $[H_4W_{22}O_{74}]^{12-}$, $[H_{10}W_{34}O_{116}]^{18-}$, or the like.

Furthermore, in an isopolyoxotungstate mixture, the number of $B^{n+}$s (sulfonium cations or iodonium cations) bonded to one isopolyoxotungstic acid is not particularly limited, and, for example, two or more different isopolyoxotungstate compounds may be contained in which two or more of $B^{n+}$ selected from an integer from 1 to 18 are bonded to one isopolyoxotungstic acid. Specific examples thereof may include four $B^{n+}$s, five $B^{n+}$s, and six $B^{n+}$s bonded to one isopolyoxotungstic acid.

Furthermore, in two or more different isopolyoxotungstate compounds represented by the general formula (I) or solvates thereof, in the case where the isopolyoxotungstic acids represented by $C^{-(x+y)}$ are common to each other, a plurality of isopolyoxotungstate compounds having different numbers of $A^{m+}$s and $B^{n+}$s bonded thereto or solvates thereof are included.

The isopolyoxotungstate mixture in this case is represented by the following general formula (I'):

$$(A^{m+})_{x'/m}(B^{n+})_{y'/n}(C^{-(x'+y')}) \qquad (I')$$

[wherein

$A^{m+}$ each independently denotes $H^+$, a metal ion, or an ammonium ion;
$B^{n+}$ each independently denotes a sulfonium cation or an iodonium cation;
$C^{-(x'+y')}$ denotes an isopolyoxotungstic acid; and
m denotes an integer in the range of 1 to 5, n denotes an integer of 1 or 2, x' and x'/m are real numbers of 0 or more, y' and y'/n are real numbers of more than 0, and x'+y' are natural numbers.]

The number of sulfonium cations or iodonium cations bonded to one isopolyoxotungstic acid can be changed by appropriately adjusting the blend ratio of the raw materials of the isopolyoxotungstic acid and a sulfonium cation or an iodonium cation used in the production of an isopolyoxotungstate compound, a solvate, or a mixture thereof.

When a sulfonium cation is used as $B^{n+}$ in an isopolyoxotungstate compound or a mixture, the elemental composition of S and W contained in the isopolyoxotungstate compound or the mixture can be determined by XRF or XPS analysis.

The XRF analysis can be a known and commonly used method, and, for example, the mole ratio of S to W in the isopolyoxotungstate compound or the mixture can be determined by a calibration curve method, SQX analysis using a fundamental parameter (FP) method, or the like.

The XPS analysis can be a known and commonly used method, and, for example, the mole ratio of S to W in the isopolyoxotungstate compound or the mixture can be determined by determining Atomic% of each element based on the peak areas of S2p and W4f.

In the case where a sulfonium cation is used as $B^{n+}$, the mole ratio of S (sulfur) to W (tungsten) in the isopolyoxotungstate compound or the mixture as determined by XRF or XPS analysis is preferably S:W = 1 to 18:4 to 34, more preferably S:W = 1 to 8:4 to 12, still more preferably S:W=1 to 8:7 to 12, particularly preferably S:W=1 to 7:8 to 12.

In the case where an iodonium cation is used as $B^{n+}$ in the isopolyoxotungstate compound or the mixture, the elemental composition of I and W contained in the isopolyoxotungstate compound or the mixture can be determined by XRF or XPS analysis.

The XRF analysis can be a known and commonly used method, and, for example, the mole ratio of I to W in the isopolyoxotungstate compound or the mixture can be determined by a calibration curve method, SQX analysis using the

fundamental parameter (FP) method, or the like.

The XPS analysis can be a known and commonly used method, and, for example, the mole ratio of S to W in the isopolyoxotungstate compound or the mixture can be determined by determining Atomic% of each element based on the peak areas of I3d and W4f.

In the case where an iodonium cation is used as $B^{n+}$, the mole ratio of I (iodine) to W (tungsten) in the isopolyoxotungstate compound or the mixture determined by XRF or XPS analysis is preferably I:W = 1 to 18:4 to 34, more preferably I:W = 1 to 8:4 to 12, still more preferably I:W = 1 to 8:7 to 12, particularly preferably I:W=1 to 7:8 to 12.

[1-5. Solvate]

It is generally known that when an isopolyoxotungstate is crystallized, the isopolyoxotungstate takes in a solvent used for crystallization and forms a solvate.

The solvate of the isopolyoxotungstate compound or the mixture according to the present embodiment is, for example, but not limited to, an alcohol solvate, such as a methanol solvate, an ethanol solvate, or an isopropanol solvate; a hydrate, such as a monohydrate, a dihydrate, a trihydrate, or a tetrahydrate; or the like.

[2. Method for Producing Isopolyoxotungstate Compound or Solvate Thereof]

A method for producing an isopolyoxotungstate compound or a solvate thereof according to another embodiment of the present invention is, for example, but not limited to, a salt exchange method, an ion exchange method, or the like.

[2-1. Salt Exchange Method]

A method for producing an isopolyoxotungstate compound represented by the general formula (I) or a solvate thereof includes:

reacting a compound represented by the general formula (VI-1) with a compound represented by the general formula (VI-2) to perform salt exchange.

$$(A^{m+})_{x/m}(B^{n+})_{y/n}(C^{-(x+y)}) \qquad (I)$$

$$(A^{m+})_{x/m}(A'^{m'+})_{y/m'}(C^{-(x+y)}) \qquad (VI\text{-}1)$$

$$(B^{n+})_{y/n}(Y^{-})_{y} \qquad (VI\text{-}2)$$

[wherein
$A^{m+}$ each independently denotes $H^+$, a metal ion, or an ammonium ion;
$A'^{m'+}$ each independently denotes a metal ion or an ammonium ion;
$B^{n+}$ each independently denotes a sulfonium cation or an iodonium cation;
$C^{-(x+y)}$ denotes an isopolyoxotungstic acid;
$Y^-$ denotes a monovalent counter anion; and
m and m' denote an integer in the range of 1 to 5, n denotes an integer of 1 or 2, x and x/m denote an integer, and y/m', y, and y/n denote a natural number.]

In the general formula (VI-2), for example, when n is 1, the formula is expressed as $(B^+)_y(Y^-)_y$, which means $y(B^+\cdot Y^-)$.

A method for producing an isopolyoxotungstate compound represented by the general formula (I) or a solvate thereof according to another embodiment may include:

reacting a compound represented by the general formula (VI-1) with a compound represented by the general formula (VI-2) to perform salt exchange; and
removing a compound represented by the general formula (VI-3) from the resulting compounds represented by the general formulae (I) and (VI-3),

$$(A^{m+})_{x/m}(B^{n+})_{y/n}(C^{-(x+y)}) \qquad (I)$$

$$(A^{m+})_{x/m}(A'^{m'+})_{y/m'}(C^{-(x+y)}) \qquad (VI\text{-}1)$$

$(B^{n+})_{y/n}(Y^-)_y$ (VI-2)

$(A'^{m'+})_{y/m'}(Y^-)_y$ (VI-3)

[wherein
$A^{m+}$ each independently denotes $H^+$, a metal ion, or an ammonium ion;
$A'^{m'+}$ each independently denotes a metal ion or an ammonium ion;
$B^{n+}$ each independently denotes a sulfonium cation or an iodonium cation;
$C^{-(x+y)}$ denotes an isopolyoxotungstic acid;
$Y^-$ denotes a monovalent counter anion; and
m and m' denote an integer in the range of 1 to 5, n denotes an integer of 1 or 2, x and x/m denote an integer, and y/m', y, and y/n denote a natural number.]

In the general formula (VI-3), for example, when m' is 1, the formula is expressed as $(A'^+)_y(Y^-)_y$, which means $y(A'^+ \cdot Y^-)$.

<Compound Represented by General Formula (VI-1)>

In a compound represented by the general formula (VI-1), which is one of the raw materials in a method for producing an isopolyoxotungstate compound or a solvate thereof according to the present embodiment,

$(A^{m+})_{x/m}(A'^{m'+})_{y/m'}(C^{-(x+y)})$ (VI-1)

$A^{m+}$ each independently denotes $H^+$, a metal ion, or an ammonium ion, and
$A'^{m'+}$ each independently denotes a metal ion or an ammonium ion.

From the perspective of promoting the salt exchange reaction, $A'^{m'+}$ is preferably $H^+$, $Li^+$, $Na^+$, $K^+$, $Rb^+$, $Cs^+$, $Be^{2+}$, $Mg^{2+}$, $Ca^{2+}$, $Sr^{2+}$, $Ba^{2+}$, $Co^{3+}$, $Bi^{3+}$, $Zr^{4+}$, $Hf^{4+}$, $Bi^{5+}$, $NH^{4+}$, a quaternary ammonium cation, or a combination thereof, more preferably $H^+$, Na+, K+, $Mg^{2+}$, $Ca^{2+}$, $NH^{4+}$, a quaternary ammonium cation, or a combination thereof, still more preferably $H^+$, Na+, K+, $NH^{4+}$, a quaternary ammonium cation, or a combination thereof.

A commercial product of a compound represented by the general formula (VI-1) is, for example, an ammonium metatungstate hydrate or a sodium polytungstate manufactured by Merck & Co., Inc.; or an ammonium metatungstate manufactured by Nippon Inorganic Colour & Chemical Co., Ltd., or the like.

<Compound Represented by General Formula (VI-2)>

In a compound represented by the general formula (VI-2), which is one of the raw materials in a method for producing an isopolyoxotungstate compound or a solvate thereof according to the present embodiment,

$(B^{n+})_{y/n}(Y^-)_y$ (VI-2)

$Y^-$ denotes a monovalent counter anion.

From the perspective of promoting the salt exchange reaction, $Y^-$ is preferably $Br^-$, $Cl^-$, $I^-$, $OH^-$, $CF_3SO_3^-$, $C_2F_5SO_3^-$, $C_3F_7SO_3^-$, $C_4F_9SO_3^-$, p-$CH_3(C_6H_4)SO_3^-$, $ClSO_3^-$, $ClO_4^-$, $(C_6F_5)_4B$-, $BF_4^-$, $PF_6^-$, $SbF_6^-$, $AlCl_4^-$, $BiF_6^-$, or $AsF_6^-$, more preferably $Br^-$, $Cl^-$, $I^-$, $OH^-$, $CF_3SO_3^-$, $C_4F_9SO_3^-$, $BF_4^-$, $PF_6^-$, or $SbF_6^-$, still more preferably $Br^-$, $Cl^-$, $CF_3SO_3^-$, $BF_4^-$, $PF_6^-$, or $SbF_6^-$.

A commercial product of a compound represented by the general formula (VI-2) is, for example, WPAG-336 (diphenyl-4-methylphenylsulfonium trifluoromethanesulfonate), WPAG-367 (diphenyl-2,4,6-trimethylphenylsulfonium p-toluenesulfonate), WPAG-370 (diphenyl(4-methoxyphenyl)sulfonium trifluoromethanesulfonate), WPAG-469 (4-methylphenyldiphenylsulfonium nonafluorobutanesulfonate), or WPAG-638 (tris(4-methylphenyl)sulfonium nonafluorobutanesulfonate) each manufactured by Fujifilm Wako Pure Chemical Corporation; or benzyl(4-hydroxyphenyl)methylsulfonium hexafluoroantimonate, diphenyl(methyl)sulfonium tetrafluoroborate, trimethylsulfonium bromide, 4-hydroxyphenyldimethylsulfonium methylsulfate, (2-bromoethyl)diphenylsulfonium trifluoromethanesulfonate, triphenylsulfonium bromide, trimethylsulfonium hydroxide, triphenylsulfonium nonafluoro-1-butanesulfonate, triphenylsulfonium hexafluorophosphate, triphenylsulfonium hexafluoroantimonate, diphenyl(4-(phenylthio)phenyl)sulfonium hexafluoroantimonate, diphenyl(4-(phenylthio)phenyl)sulfonium hexafluorophosphate, (3-chloropropyl)diphenylsulfonium tetrafluoroborate, (2-carboxyethyl)dimethylsulfonium chloride, (2-carboxyethyl)dimethylsulfonium bromide, tri-p-tolylsulfonium trifluoromethanesulfonate, (difluoromethyl)bis(2,5-dimethylphenyl)sulfonium tetrafluoroborate, (4-hydroxyphenyl)methyl(2-methylbenzyl)sulfonium hexafluoroantimonate, tributylsulfonium iodide, (thiodi-4,1-phenylene)bis(diphenylsulfonium) bis(hexafluorophosphate), 4-isopropyl-4'-methyldiphenyliodonium tetrakis(pentafluorophenyl)borate, (4-(bromomethyl)phenyl)(2,4,6-trimethoxyphenyl)iodonium p-toluenesulfonate, 4-biphenylyl(2,4,6-trimethoxyphenyl)iodonium trifluoromethanesulfonate, bis(4-t-butylphenyl)iodonium hexafluorophosphate, bis(2,4,6-trimethylphenyl)iodonium trifluoromethanesulfonate, bis(2,4,6-trimethylpyridine)iodonium hexafluorophosphate, bis(4-fluorophenyl)iodonium trifluoromethanesulfonate, ethynyl(phenyl)iodonium tetrafluoroborate, (4-(trifluoromethyl)phenyl)(2,4,6-trimethoxyphenyl)iodonium p-toluenesulfonate, (4-(trifluoromethyl)phenyl)(2,4,6-trimethylphenyl)iodonium trifluoromethanesulfonate, diphenyliodonium chloride, diphenyliodonium bromide, diphenyliodonium hexafluorophosphate, or diphenyliodonium trifluoromethanesulfonate each manufactured by Tokyo Chemical Industry Co., Ltd., or the like.

<Blend Mole Ratio of Compound Represented by General Formula (VI-1) to Compound Represented by General Formula (VI-2)>

In a reaction of a compound represented by the general formula (VI-1) and a compound represented by the general formula (VI-2), the blend mole ratio of the compound represented by the general formula (VI-1) to the compound represented by the general formula (VI-2) is not particularly limited and can be appropriately determined according to the mole ratio of $A^{m+}$ to $B^{n+}$ contained in the target isopolyoxotungstate or a solvate thereof.
The blend mole ratio of a compound represented by the general formula (VI-1) to a compound represented by the general formula (VI-2), the compound represented by the general formula (VI-1):the compound represented by the general formula (VI-2), is preferably 1 to 3:1 to 20, more preferably 1 to 3:1 to 18, still more preferably 2:1 to 15.

<Reaction between Compound Represented by General Formula (VI-1) and Compound Represented by General Formula (VI-2)>

The reaction conditions of a compound represented by the general formula (VI-1) and a compound represented by the general formula (VI-2) are not particularly limited and can be known and commonly used reaction conditions, and the type of solvent, the reaction temperature, the reaction time, the pressure, and the like can be appropriately determined.
The type of solvent is, for example, but not limited to, water, methanol, ethanol, 1,4-dioxane, acetonitrile, 1-propanol, 2-propanol, ethyl acetate, acetonitrile, acetone, dichloromethane, chloroform, dimethylformamide, diethyl ether, or the like. These solvents may be used alone or in combination.
The reaction temperature is, for example, but not limited to, room temperature or a high temperature.
The reaction time is not particularly limited and is preferably 1 minute to 24 hours, more preferably 1 minute to 5 hours.
The pressure is not particularly limited and may be atmospheric pressure or a high pressure.

<Step of Removing Compound Represented by General Formula (VI-3)>

A method of removing a compound represented by the general formula (VI-3) is, for example, but not limited to, a method of removing a compound represented by the general formula (VI-3) formed as a by-product by extraction into an aqueous layer, a method of precipitation and filtration as an inorganic salt, a method of crystallization and filtration of a target compound represented by the general formula (I) or a solvate thereof, or the like.

$$(A'^{m'+})_{y/m'}(Y^-)_y \qquad (VI\text{-}3)$$

$$(A^{m+})_{x/m}(B^{n+})_{y/n}(C^{-(x+y)}) \qquad (I)$$

The method of crystallization and filtration of a target compound represented by the general formula (I) or a solvate thereof is, for example, but not limited to, a method of heating and dissolving the target compound in a solvent and then precipitating crystals by slow cooling, a method of combining a poor solvent and a good solvent for crystallization, or the like.
The poor solvent can be appropriately determined for the target compound and is, for example, diethyl ether, dipropyl ether, ethyl acetate, butyl acetate, hexane, toluene, 2-propanol, ethanol, methanol, or the like.
The good solvent can be appropriately determined for the target compound and is, for example, water, methanol, ethanol, 1,4-dioxane, dimethylformamide, chloroform, dichloromethane, or the like.

[2-2. Ion Exchange Method]

A method for producing an isopolyoxotungstate compound represented by the general formula (I) or a solvate

thereof according to another embodiment includes:

ion-exchanging $A'^{m'+}$ of a compound represented by the general formula (VI-1) with $H^+$ using an ion-exchange resin; and

reacting a compound represented by the general formula (VI-4) produced by the above step with a compound represented by the general formula (VI-2).

$$(A^{m+})_{x/m}(B^{n+})_{y/n}(C^{-(x+y)}) \qquad (I)$$

$$(A^{m+})_{x/m}(A'^{m'+})_{y/m'}(C^{-(x+y)}) \qquad (VI\text{-}1)$$

$$(A^{m+})_{x/m}(H^{+})_{y}(C^{-(x+y)} \qquad (VI\text{-}4)$$

$$(B^{n+})_{y/n}(Y^{-})_{y} \qquad (VI\text{-}2)$$

[wherein
$A^{m+}$ each independently denotes $H^+$, a metal ion, or an ammonium ion;
$A'^{m'+}$ each independently denotes a metal ion or an ammonium ion;
$B^{n+}$ each independently denotes a sulfonium cation or an iodonium cation;
$C^{-(x+y)}$ denotes an isopolyoxotungstic acid;
$Y^-$ denotes a monovalent counter anion; and
m and m' denote an integer in the range of 1 to 5, n denotes an integer of 1 or 2, x and x/m denote an integer, and y/m', y, and y/n denote a natural number.]

<Ion-Exchange Resin>

The ion-exchange resin is not particularly limited as long as $A'^{m'+}$ of a compound represented by the general formula (VI-1) can be ion-exchanged with $H^+$ and can be a known and commonly used ion-exchange resin.

The ion-exchange resin is, for example, but not limited to, a strongly acidic cation-exchange resin, a weakly acidic cation-exchange resin, or the like.

Examples of commercial products of the ion-exchange resin include Amberlite (TM) IR-200CT, HPR1200 H, HPR1024 H, HPR1006NNC H, and Dowex (TM) 50W each manufactured by Dow Chemical; ORLITE (TM) DS-1 and DS-4 manufactured by Organo Corporation; and the like.

A method of ion-exchanging $A'^{m'+}$ of a compound represented by the general formula (VI-1) with $H^+$ using an ion-exchange resin can be a known and commonly used method.

The method is, for example, but not limited to, a method of filling a column with an ion-exchange resin, then passing a solution, such as water or an organic solvent, containing a compound represented by the general formula (VI-1) through the column to bring the compound represented by the general formula (VI-1) and the ion-exchange resin into contact with each other, and performing elution with an eluting solvent, a method of mixing an ion-exchange resin with a compound represented by the general formula (VI-1) in a beaker and then removing the ion-exchange resin by filtration, or the like.

The eluting solvent that can be used to pass the solution containing a compound represented by the general formula (VI-1) through a column filled with an ion-exchange resin is, for example, but not limited to, Milli-Q water, pure (ion-exchanged water, demineralized water), methanol, ethanol, or the like.

The space velocity (SV, $h^{-1}$) at which a compound represented by the general formula (VI-1) is brought into contact using a column filled with an ion-exchange resin is not particularly limited as long as $A'^{m'+}$ of the compound represented by the general formula (VI-1) can be ion-exchanged with $H^+$. The SV preferably ranges from 0.1 to 100, more preferably 0.5 to 50, still more preferably 1 to 30. The SV is a unit indicating how many times as much liquid as the resin volume is passed per hour.

<Reaction with Compound Represented by General Formula (VI-2)>

In a reaction of a compound represented by the general formula (VI-4) produced by ion-exchanging $A'^{m'+}$ of a compound represented by the general formula (VI-1) with $H^+$ and a compound represented by the general formula (VI-2), the reaction conditions, the blend ratio of both, and the like can be appropriately determined within the range of the reaction conditions, the blend ratio, and the like similar to those in the salt exchange method.

$$(A^{m+})_{x/m}(H^{+})_{y}(C^{-(x+y)} \qquad (VI\text{-}4)$$

$(B^{n+})_{y/n}(Y^-)_y$ (VI-2)

<Step of Removing By-Product>

A reaction between a compound represented by the general formula (VI-4) and a compound represented by the general formula (VI-2) yields an isopolyoxotungstate compound represented by the general formula (I) and $yH^+Y^-$ as a by-product. A method of removing the by-product can be the same as the method of removing a compound represented by the general formula (VI-3) in the salt exchange method.

**EXAMPLES**

The present invention will be more specifically described below with reference to Examples, but the present invention is not limited to these Examples.

[Example 1: Hexakis(bis(2-trifluoromethylphenyl)phenylsulfonium)metatungstate]

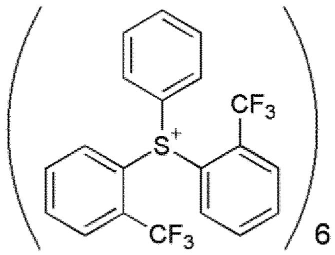

$[H_2W_{12}O_{40}]^{6-}$

<Salt Exchange Method>

6.68 g of bis(2-trifluoromethylphenyl)phenylsulfonium chloride was dissolved in 99.36 g of pure water, 6.88 g of an ammonium metatungstate (manufactured by Nippon Inorganic Colour & Chemical Co., Ltd.: an ammonium metatungstate hydrate ($(NH_4)_6[H_2W_{12}O_{40}]\cdot xH_2O$)) was added thereto, and the reaction liquid was stirred at room temperature for 30 minutes. A powder produced by filtering the reaction liquid was dried under reduced pressure at room temperature for 18 hours. The dried powder was dissolved in 100 g of dimethylformamide, 335 g of methanol was then added thereto, and a target compound was produced by crystallization at room temperature.

$^1$H-NMR (400 MHz, DMSO-D6): $\delta$ (ppm) = 5.97 (s, 0.33H), 7.65-8.35 (m, 13H).
$^{183}$W-NMR (20.84 MHz, DMSO-D6): $\delta$ (ppm) = -100.13 (s, 12W).
ESI-MS: POSITIVE m/z 399.1 ($[C_{20}H_{13}F_6S]^+$)
NEGATIVE m/z 712.3 (median) ($[H_4W_{12}O_{40}]^{4-}$)

<Ion Exchange Method>

60 g of an ammonium metatungstate was dissolved in 240 g of ion-exchanged water to prepare aqueous ammonium metatungstate. A column with an inner diameter of 30 mm was filled with 60 $cm^3$ of a strongly acidic cation-exchange resin ORLITE (TM) DS-1 (Organo Corporation). The aqueous ammonium metatungstate was passed through the column at a rate in the range of approximately 1 to 15 $cm^3$/min, and ion-exchanged water was then passed through the column at the same rate. Among the eluates, an eluate with an acidic pH was collected.

35 g of bis(2-trifluoromethylphenyl)phenylsulfonium chloride was added to the collected eluate, and the reaction liquid was stirred at room temperature for 30 minutes. A white powder produced by filtering the reaction liquid was dried under reduced pressure at room temperature for 18 hours. The dried white powder was dissolved in 30 g of dimethylformamide, methanol was then added thereto, and a target compound was produced by crystallization at room temperature.

[Example 2: Hexakis(triphenylsulfonium)metatungstate]

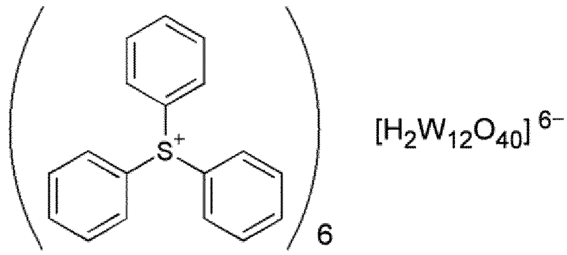

A target compound was produced in the same manner as in the salt exchange method in Example 1 except that triphenylsulfonium chloride was used as a raw material compound instead of the bis(2-trifluoromethylphenyl)phenylsulfonium chloride.

$^{1}$H-NMR (400 MHz, DMSO-D6): δ (ppm) = 7.74-8.35 (m, 15H), 5.97 (s, 0.33H).
$^{183}$W-NMR (20.84 MHz, DMSO-D6): δ (ppm) = -100.13 (s, 12W).
ESI-MS: POSITIVE m/z 263.1 ($[C_{18}H_{15}S]^+$)
NEGATIVE m/z 712.3 (median) ($[H_4W_{12}O_{40}]^{4-}$)

[Example 3: Hexakis(bis(4-t-butylphenyl)iodonium)metatungstate]

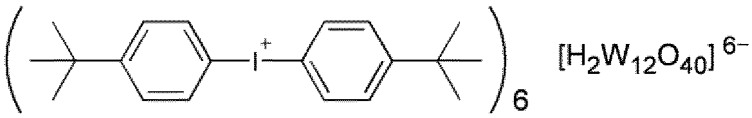

A target compound was produced in the same manner as in the salt exchange method in Example 1 except that bis(4-t-butylphenyl)iodonium methylsulfate was used as a raw material compound instead of the bis(2-trifluoromethylphenyl)phenylsulfonium chloride.

$^{1}$H-NMR (400 MHz, DMSO-D6): δ (ppm) = 1.22 (s, 18H), 5.97 (s, 0.33H), 7.51 (d, 4H), 8.19 (d, 4H).
$^{183}$W-NMR (20.84 MHz, DMSO-D6): δ (ppm) = -95.39 (s, 12W).
ESI-MS: POSITIVE m/z 393.1 ($[C_{20}H_{26}I]^+$)
NEGATIVE m/z 712.3 (median) ($[H_4W_{12}O_{40}]^{4-}$)

[Example 4: isopolyoxotungstate mixture]

A target compound was produced in the same manner as in the salt exchange method in Example 1 except that the amount of bis(2-trifluoromethylphenyl)phenylsulfonium chloride used as a raw material compound was decreased from 6.68 g to 6.02 g.

<XRF Analysis>

The elemental composition of S and W contained in the produced target compound was analyzed by X-ray fluorescence analysis (XRF analysis). An X-ray fluorescence spectrometer (manufactured by Rigaku Corporation: ZSXPrimus) was used in the XRF analysis to calculate the elemental ratio of S to W by SQX analysis using the fundamental parameter (FP) method. Under the analytical conditions of the FP method, qualitative analysis was performed at a tube voltage of 50 kV, at a tube current of 50mA, and using a step scan (step: 0.05 degrees, measurement time: 0.4 seconds), and a quantitative value was obtained by SQF analysis for an identified peak. The results are described below.

S (mass%):W (mass%) = 1.00:13.01

S (mass%/atomic weight (32.07)):W (mass%/atomic weight (183.8)) = 1.00:2.27

The results show that in the produced target compound, 5.29 mol of bis(2-trifluoromethylphenyl)phenylsulfonium cation is bonded to 1 mol of $[H_2W_{12}O_{40}]^{6-}$.

<XPS Analysis>

The elemental composition of W, S, C, N, O, and F contained in the produced target compound was quantitatively analyzed by X-ray photoelectron spectroscopy (XPS).

Using K-ALPHA manufactured by Thermofisher Scientific as an XPS analyzer, Atomic% of each element was determined based on the peak areas of W4f, S2p, C1s, N1s, O1s, and F1s under the following conditions, and the results are shown in Table 1.

X-ray source: monochromated AlK$\alpha$ radiation
Voltage: 15 kV
Beam diameter: 100 $\mu$m
Photoelectron take-off angle: 45 degrees
Scan range: 15.5 to 1100 eV

[Table 1]

| Element | W | S | C | N | O | F | Total |
|---|---|---|---|---|---|---|---|
| Atomic % | 5.7 | 2.51 | 53.12 | 0.54 | 20.02 | 18.1 | 99.99 |

The results of the quantitative analysis by the XPS show that in the produced target compound, 5.28 mol of bis(2-trifluoromethylphenyl)phenylsulfonium cation is bonded to 1 mol of $[H_2W_{12}O_{40}]^{6-}$.

From the mole ratio of the sulfonium cation bonded to the isopolyoxotungstic acid, it can be seen that the mole ratio of the sulfonium cation bonded to the isopolyoxotungstic acid can be adjusted by adjusting the blend ratio of both in the salt exchange method or the ion exchange method.

[Example 5: Hexakis(tributylsulfonium)metatungstate]

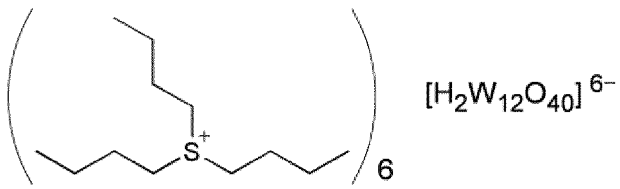

A target compound was produced in the same manner as in the salt exchange method in Example 1 except that tributylsulfonium chloride was used as a raw material compound instead of the bis(2-trifluoromethylphenyl)phenylsulfonium chloride.

$^1$H-NMR (400 MHz, DMSO-D6): $\delta$ (ppm) = 0.81-0.93 (m, 9H), 1.35-1.44 (m, 6H), 1.68 (quint, 6H), 3.36 (t, 6H), 5.97 (s, 0.33H).
$^{183}$W-NMR (20.84 MHz, DMSO-D6): $\delta$ (ppm) = -100.13 (s, 12W).
ESI-MS: POSITIVE m/z 203.2 ($[C_{12}H_{27}S]^+$)
NEGATIVE m/z 712.3 (median) ($[H_4W_{12}O_{40}]^{4-}$)

[Example 6: Hexakis(1-phenylthian-1-ium)metatungstate]

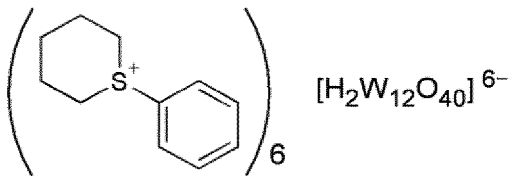

A target compound was produced in the same manner as in the salt exchange method in Example 1 except that 1-phenylthian-1-ium triflate was used as a raw material compound instead of the bis(2-trifluoromethylphenyl)phenylsulfonium chloride.

$^1$H-NMR (400 MHz, DMSO-D6): δ (ppm) = 1.66-2.16 (m, 6H), 3.79-3.83 (m, 4H), 5.97 (s, 0.33H), 7.75-7.76 (m, 3H), 8.05 (d, 2H).
$^{183}$W-NMR (20.84 MHz, DMSO-D6): δ (ppm) = -100.13 (s, 12W).
ESI-MS: POSITIVE m/z 179.1 ($[C_{11}H_{15}S]^+$)
NEGATIVE m/z 712.3 (median) ($[H_4W_{12}O_{40}]^{4-}$)

[Example 7: Hexakis(4-(4-t-butylphenyl)-1,4-oxathian-4-ium)metatungstate]

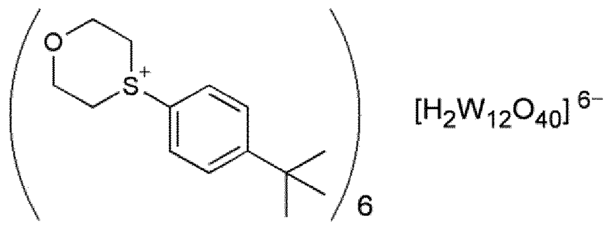

A target compound was produced in the same manner as in the salt exchange method in Example 1 except that 4-(4-t-butylphenyl)-1,4-oxathian-4-ium triflate was used as a raw material compound instead of the bis(2-trifluoromethylphenyl)phenylsulfonium chloride.

$^1$H-NMR (400 MHz, DMSO-D6): δ (ppm) = 1.32 (s, 9H), 2.49-2.79 (m, 4H), 3.80-3.95 (m, 4H), 5.97 (s, 0.33H), 7.75 (dd, 2H), 7.98 (d, 2H).
$^{183}$W-NMR (20.84 MHz, DMSO-D6): δ (ppm) = -100.13 (s, 12W).
ESI-MS: POSITIVE m/z 237.1 ($[C_{14}H_{21}OS]^+$)
NEGATIVE m/z 712.3 (median) ($[H_4W_{12}O_{40}]^{4-}$)

[Example 8: Hexakis(1-(4-(2-methoxyethoxy)naphthalene-1-yl)tetrahydrothiophen-1-ium)metatungstate]

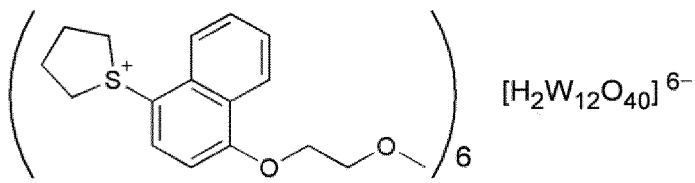

A target compound was produced in the same manner as in the salt exchange method in Example 1 except that 1-(4-(2-methoxyethoxy)naphthalene-1-yl)tetrahydrothiophen-1-ium triflate was used as a raw material compound instead of the bis(2-trifluoromethylphenyl)phenylsulfonium chloride.

$^1$H-NMR (400 MHz, DMSO-D6): δ (ppm) = 2.30-2.45 (m, 4H), 3.38 (s, 3H), 3.74-3.85 (m, 4H), 4.00-4.09 (m, 2H), 4.43 (t, 2H), 5.97 (s, 0.33H), 7.23 (d, 1H), 7.73-7.89 (m, 2H), 8.11 (d, 1H), 8.31-8.36 (m, 2H).
$^{183}$W-NMR (20.84 MHz, DMSO-D6): δ (ppm) = -100.13 (s, 12W).
ESI-MS: POSITIVE m/z 289.1 ($[C_{17}H_{21}O_2S]^+$)

NEGATIVE m/z 712.3 (median) ($[H_4W_{12}O_{40}]^{4-}$)

[Example 9: Hexakis(1-(2-((2-methyladamantane-2-yl)oxy)-2-oxoethyl)tetrahydrothiophen-1-ium)metatungstate]

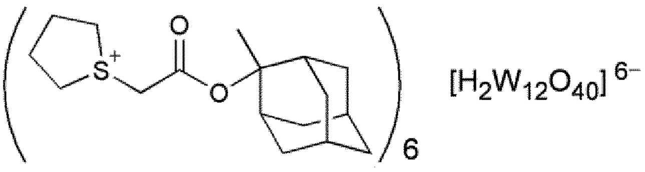

A target compound was produced in the same manner as in the salt exchange method in Example 1 except that 1-(2-((2-methyladamantane-2-yl)oxy)-2-oxoethyl)tetrahydrothiophen-1-ium triflate was used as a raw material compound instead of the bis(2-trifluoromethylphenyl)phenylsulfonium chloride.

$^1$H-NMR (400 MHz, DMSO-D6): δ (ppm) = 1.56-2.33 (m, 21H), 3.38-3.58 (m, 4H), 4.46 (s, 2H), 5.97 (s, 0.33H).
$^{183}$W-NMR (20.84 MHz, DMSO-D6): δ (ppm) = -100.13 (s, 12W).
ESI-MS: POSITIVE m/z 295.2 ($[C_{17}H_{27}O_2S]^+$)
NEGATIVE m/z 712.3 (median) ($[H_4W_{12}O_{40}]^{4-}$)

[Example 10: Hexakis(1-(4-(t-butyl)phenyl)benzo[b]thiophen-1-ium)metatungstate]

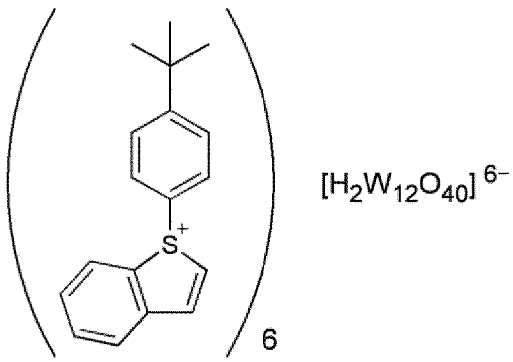

A target compound was produced in the same manner as in the salt exchange method in Example 1 except that 1-(4-(t-butyl)phenyl)benzo[b]thiophen-1-ium triflate was used as a raw material compound instead of the bis(2-trifluoromethylphenyl)phenylsulfonium chloride.

$^1$H-NMR (400 MHz, DMSO-D6): δ (ppm) = 8.28 (d, 2H, ArH), 8.12 (d, 1H, ArH), 7.88 (t, 1H, ArH), 7.80 (d, 1H), 7.62-7.74 (m, 5H), 5.97 (s, 0.33H), 1.27 (s, 9H).
$^{183}$W-NMR (20.84 MHz, DMSO-D6): δ (ppm) = -100.13 (s, 12W).
ESI-MS: POSITIVE m/z 267.4 ($[C_{18}H_{19}OS]^+$)
NEGATIVE m/z 712.3 (median) ($[H_4W_{12}O_{40}]^{4-}$)

[Example 11: Pentakis((4-((diisopropylcarbamoyl)oxy)phenyl)dimethylsulfonium)metatungstate]

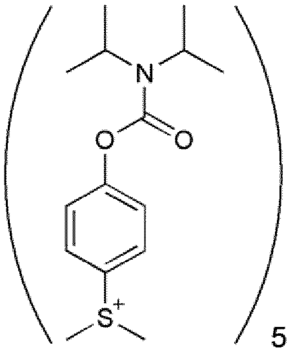

$[H_3W_{12}O_{40}]^{5-}$

A target compound was produced in the same manner as in the salt exchange method in Example 1 except that (4-((diisopropylcarbamoyl)oxy)phenyl)dimethylsulfonium triflate was used as a raw material compound instead of the bis(2-trifluoromethylphenyl)phenylsulfonium chloride.

$^1$H-NMR (400 MHz, DMSO-D6): δ (ppm) = 1.21 (d, 12H), 3.18 (m, 2H), 3.26 (s, 6H), 6.77 (s, 0.60H), 7.47-7.50 (m, 2H), 8.08-8.10 (m, 2H).
$^{183}$W-NMR (20.84 MHz, DMSO-D6): δ (ppm) = -87.59 (s, 12W).
ESI-MS: POSITIVE m/z 282.2 ($[C_{15}H_{24}NO_2S]^+$)
NEGATIVE m/z 712.3 (median) ($[H_4W_{12}O_{40}]^{4-}$)

[Example 12: Pentakis(5-(3-(trifluoromethyl)phenyl)dibenzo[b,d]thiophen-5-ium)metatungstate]

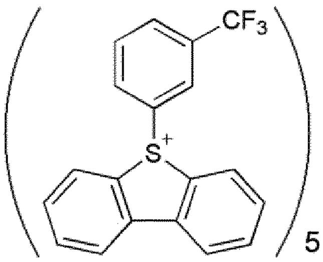

$[H_3W_{12}O_{40}]^{5-}$

A target compound was produced in the same manner as in the salt exchange method in Example 1 except that 5-(3-(trifluoromethyl)phenyl)dibenzo[b,d]thiophen-5-ium chloride was used as a raw material compound instead of the bis(2-trifluoromethylphenyl)phenylsulfonium chloride.

$^1$H-NMR (400 MHz, DMSO-D6): δ (ppm) = 6.77 (s, 0.60H), 7.35 (d, 1H), 7.65-7.83 (m, 3H), 7.95 (t, 2H), 8.05 (d, 1H), 8.40 (d, 2H), 8.55 (d, 2H), 8.63 (s, 1H).
$^{183}$W-NMR (20.84 MHz, DMSO-D6): δ (ppm) = -87.59 (s, 12W).
ESI-MS: POSITIVE m/z 329.1 ($[C_{19}H_{12}F_3S]^+$)
NEGATIVE m/z 712.3 (median) ($[H_4W_{12}O_{40}]^{4-}$)

[Example 13: Pentakis(triphenylsulfonium)metatungstate]

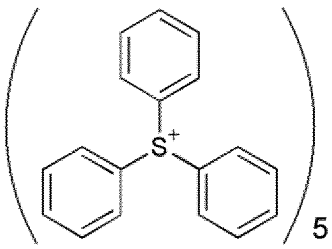

$[H_3W_{12}O_{40}]^{5-}$

A target compound was produced in the same manner as in the salt exchange method in Example 1 except that triphenylsulfonium chloride was used as a raw material compound instead of the bis(2-trifluoromethylphenyl)phenylsulfonium chloride.

> $^1$H-NMR (400 MHz, DMSO-D6): δ (ppm) = 7.78-7.87 (m, 15H), 6.77 (s, 0.60H).
> $^{183}$W-NMR (20.84 MHz, DMSO-D6): δ (ppm) = -87.59 (s, 12W).
> ESI-MS: POSITIVE m/z 263.1 ($[C_{18}H_{15}S]^+$)
> NEGATIVE m/z 712.3 (median) ($[H_4W_{12}O_{40}]^{4-}$)

[Example 14: Hexakis(phenyl bis(1-naphthyl)sulfonium)metatungstate]

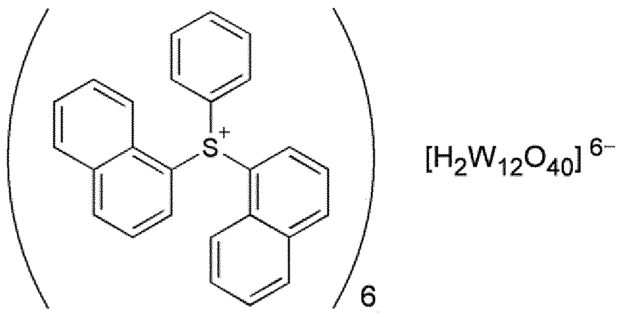

A target compound was produced in the same manner as in the salt exchange method in Example 1 except that phenyl bis(1-naphthyl)sulfonium triflate was used as a raw material compound instead of the bis(2-trifluoromethylphenyl) phenylsulfonium chloride.

> $^1$H-NMR (400 MHz, DMSO-D6): δ (ppm) = 5.97 (s, 0.33H), 7.55 (d, 2H), 7.82-7.88 (m, 8H), 7.93-7.97 (m, 1H), 8.03 (d, 2H), 8.32 (d, 2H), 8.38 (d, 2H), 8.55 (d, 2H).
> $^{183}$W-NMR (20.84 MHz, DMSO-D6): δ (ppm) = -100.13 (s, 12W).
> ESI-MS: POSITIVE m/z 363.1 ($[C_{26}H_{19}S]^+$)
> NEGATIVE m/z 712.3 (median) ($[H_4W_{12}O_{40}]^{4-}$)

[Example 15: Hexakis(diphenyl(p-tolyl)sulfonium)metatungstate]

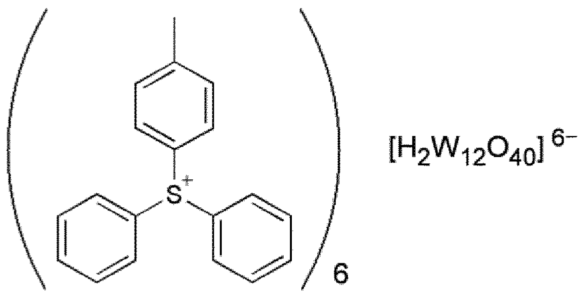

A target compound was produced in the same manner as in the salt exchange method in Example 1 except that diphenyl(p-tolyl)sulfonium chloride was used as a raw material compound instead of the bis(2-trifluoromethylphenyl) phenylsulfonium chloride.

> $^1$H-NMR (400 MHz, DMSO-D6): δ (ppm) = 7.72-7.84 (m, 12H), 7.56 (d, 2H), 5.97 (s, 0.33H), 2.49 (s, 3H).
> $^{183}$W-NMR (20.84 MHz, DMSO-D6): δ (ppm) = -100.13 (s, 12W).
> ESI-MS: POSITIVE m/z 277.4 ($[C_{19}H_{17}S]^+$)
> NEGATIVE m/z 712.3 (median) ($[H_4W_{12}O_{40}]^{4-}$)

[Example 16: Hexakis((4-hydroxy-3,5-dimethylphenyl)diphenylsulfonium)metatungstate]

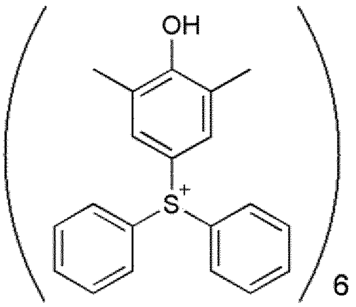

$[H_2W_{12}O_{40}]^{6-}$

A target compound was produced in the same manner as in the salt exchange method in Example 1 except that (4-hydroxy-3,5-dimethylphenyl)diphenylsulfonium methanesulfonate was used as a raw material compound instead of the bis(2-trifluoromethylphenyl)phenylsulfonium chloride.

$^1$H-NMR (400 MHz, DMSO-D6): $\delta$ (ppm) = 10.05 (s, 1H), 7.64.-7.87 (m, 10H), 7.56 (s, 2H), 5.97 (s, 0.33H), 2.22 (m, 6H).
$^{183}$W-NMR (20.84 MHz, DMSO-D6): $\delta$ (ppm) = -100.13 (s, 12W).
ESI-MS: POSITIVE m/z 307.4 ($[C_{20}H_{19}S]^+$)
NEGATIVE m/z 712.3 (median) ($[H_4W_{12}O_{40}]^{4-}$)

[Example 17: Pentakis(tris(3,5-difluorophenyl)sulfonium)metatungstate]

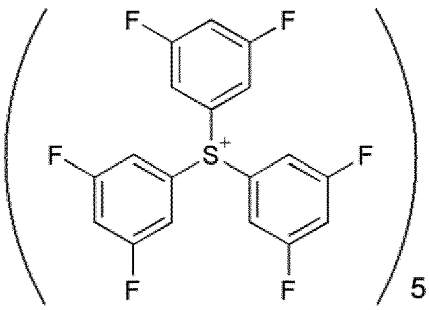

$[H_3W_{12}O_{40}]^{5-}$

A target compound was produced in the same manner as in the salt exchange method in Example 1 except that tris(3,5-difluorophenyl)sulfonium triflate was used as a raw material compound instead of the bis(2-trifluoromethylphenyl) phenylsulfonium chloride.

$^1$H-NMR (400 MHz, DMSO-D6): $\delta$ (ppm) = 6.77 (s, 0.60H), 7.82-7.96 (m, 9H).
$^{183}$W-NMR (20.84 MHz, DMSO-D6): $\delta$ (ppm) = -87.59 (s, 12W).
ESI-MS: POSITIVE m/z 371.0 ($[C_{18}H_9F_6S]^+$)
NEGATIVE m/z 712.3 (median) ($[H_4W_{12}O_{40}]^{4-}$)

[Example 18: Hexakis(diphenyl(4-(phenylthio)phenyl)sulfonium)metatungstate]

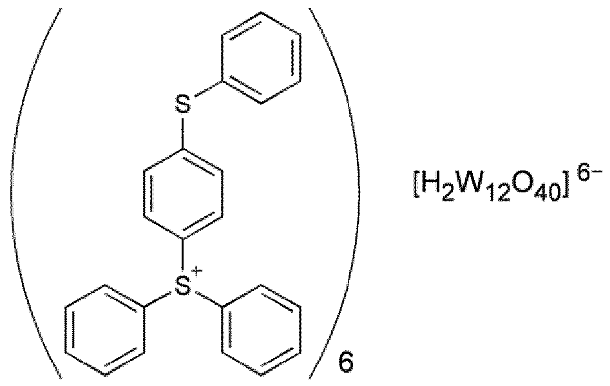

A target compound was produced in the same manner as in the salt exchange method in Example 1 except that diphenyl(4-(phenylthio)phenyl)sulfonium methanesulfonate was used as a raw material compound instead of the bis(2-trifluoromethylphenyl)phenylsulfonium chloride.

$^{1}$H-NMR (400 MHz, DMSO-D6): δ (ppm) = 7.39 (d, 2H), 7.51-7.62 (m, 5H), 7.73-7.85 (m, 12H), 5.97 (s, 0.33H).
$^{183}$W-NMR (20.84 MHz, DMSO-D6): δ (ppm) = -100.13 (s, 12W).
ESI-MS: POSITIVE m/z 371.1 ($[C_{24}H_{19}S_2]^+$)
NEGATIVE m/z 712.3 (median) ($[H_4W_{12}O_{40}]^{4-}$)

[Example 19: Hexakis((4-(cyclohexylsulfonyl)phenyl)diphenylsulfonium)metatungstate]

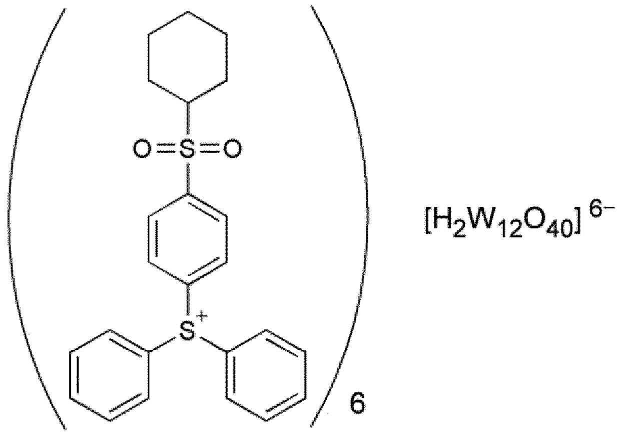

A target compound was produced in the same manner as in the salt exchange method in Example 1 except that (4-(cyclohexylsulfonyl)phenyl)diphenylsulfonium triflate was used as a raw material compound instead of the bis(2-trifluoromethylphenyl)phenylsulfonium chloride.

$^{1}$H-NMR (400 MHz, DMSO-D6): δ (ppm) = 1.09-2.05 (m, 10H), 3.30-3.45 (m, 1H), 5.97 (s, 0.33H), 7.71-8.21 (m, 14H).
$^{183}$W-NMR (20.84 MHz, DMSO-D6): δ (ppm) = -100.13 (s, 12W).
ESI-MS: POSITIVE m/z 409.1 ($[C_{24}H_{25}O_2S_2]^+$)
NEGATIVE m/z 712.3 (median) ($[H_4W_{12}O_{40}]^{4-}$)

[Example 20: Pentakis((4-((adamantane-2-carbonyl)oxy)-3,5-dimethylphenyl)diphenylsulfonium)metatungstate]

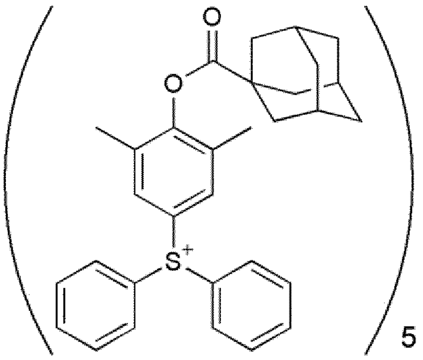

$[H_3W_{12}O_{40}]^{5-}$

A target compound was produced in the same manner as in the salt exchange method in Example 1 except that (4-((adamantane-2-carbonyl)oxy)-3,5-dimethylphenyl)diphenylsulfonium methanesulfonate was used as a raw material compound instead of the bis(2-trifluoromethylphenyl)phenylsulfonium chloride.

$^1$H-NMR (400 MHz, DMSO-D6): δ (ppm) = 1.66-2.03 (m, 15H), 2.13 (s, 6H), 6.77 (s, 0.60H), 7.69 (s, 2H), 7.76-7.87 (m, 10H).
$^{183}$W-NMR (20.84 MHz, DMSO-D6): δ (ppm) = -87.59 (s, 12W).
ESI-MS: POSITIVE m/z 469.2 ($[C_{31}H_{33}O_2S]^+$)
NEGATIVE m/z 712.3 (median) ($[H_4W_{12}O_{40}]^{4-}$)

[Example 21: Pentakis((3,5-dimethyl-4-(2-((2-methyladamantane-2-yl)oxy)-2-oxoethoxy)phenyl)diphenylsulfonium) metatungstate]

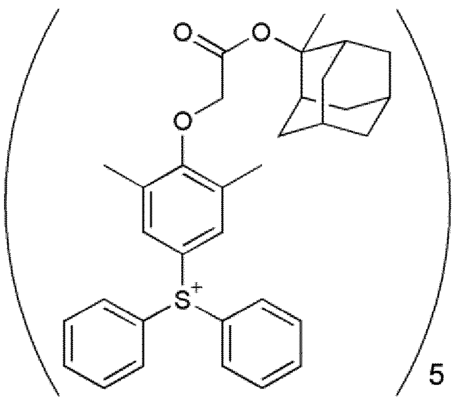

$[H_3W_{12}O_{40}]^{5-}$

A target compound was produced in the same manner as in the salt exchange method in Example 1 except that (3,5-dimethyl-4-(2-((2-methyladamantane-2-yl)oxy)-2-oxoethoxy)phenyl)diphenylsulfonium bromide was used as a raw material compound instead of the bis(2-trifluoromethylphenyl)phenylsulfonium chloride.

$^1$H-NMR (400 MHz, DMSO-D6): δ (ppm) = 1.49-1.97 (m, 17H), 2.31 (s, 6H), 4.62 (s, 2H), 6.77 (s, 0.60H), 7.61 (s, 2H), 7.75-7.86 (m, 10H).
$^{183}$W-NMR (20.84 MHz, DMSO-D6): δ (ppm) = -87.59 (s, 12W).
ESI-MS: POSITIVE m/z 513.2 ($[C_{33}H_{37}O_3S]^+$)
NEGATIVE m/z 712.3 (median) ($[H_4W_{12}O_{40}]^{4-}$)

[Example 22: Pentakis((4-(2-((1-ethylcyclopentyl)oxy)-2-oxoethoxy)-3,5-dimethylphenyl)diphenylsulfonium)metatungstate]

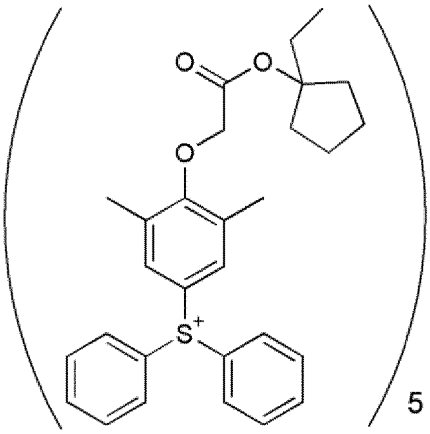

$[H_3W_{12}O_{40}]^{5-}$

A target compound was produced in the same manner as in the salt exchange method in Example 1 except that (4-(2-((1-ethylcyclopentyl)oxy)-2-oxoethoxy)-3,5-dimethylphenyl)diphenylsulfonium bromide was used as a raw material compound instead of the bis(2-trifluoromethylphenyl)phenylsulfonium chloride.

$^1$H-NMR (400 MHz, DMSO-D6): $\delta$ (ppm) = 0.77-0.81 (t, 3H), 1.48-1.75 (m, 6H), 1.90-1.93 (m, 4H), 2.29 (m, 6H), 4.55 (s, 2H), 6.77 (s, 0.60H), 7.59 (s, 2H), 7.76-7.82 (m, 10H).
$^{183}$W-NMR (20.84 MHz, DMSO-D6): $\delta$ (ppm) = -87.59 (s, 12W).
ESI-MS: POSITIVE m/z 461.2 ($[C_{29}H_{33}O_3S]^+$)
NEGATIVE m/z 712.3 (median) ($[H_4W_{12}O_{40}]^{4-}$)

[Example 23: Hexakis(dimesityliodonium)metatungstate]

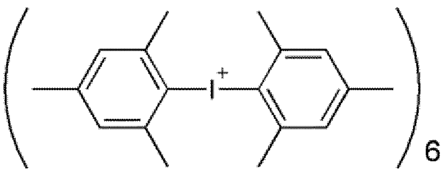

$[H_2W_{12}O_{40}]^{6-}$

A target compound was produced in the same manner as in the salt exchange method in Example 1 except that dimesityliodonium triflate was used as a raw material compound instead of the bis(2-trifluoromethylphenyl)phenylsulfonium chloride.

$^1$H-NMR (400 MHz, DMSO-D6): $\delta$ (ppm) = 2.29 (s, 6H), 2.47 (s, 12H), 5.97 (s, 0.33H), 7.19 (s, 4H).
$^{183}$W-NMR (20.84 MHz, DMSO-D6): $\delta$ (ppm) = -95.39 (s, 12W).
ESI-MS: POSITIVE m/z 365.1 ($[C_{18}H_{22}I]^+$)
NEGATIVE m/z 712.3 (median) ($[H_4W_{12}O_{40}]^{4-}$)

[Example 24: Hexakis((3-bromophenyl)(mesityl)iodonium)metatungstate]

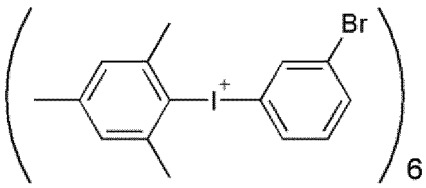

$[H_2W_{12}O_{40}]^{6-}$

A target compound was produced in the same manner as in the salt exchange method in Example 1 except that (3-bromophenyl)(mesityl)iodonium hexafluoroantimonate was used as a raw material compound instead of the bis(2-

trifluoromethylphenyl)phenylsulfonium chloride.

$^{1}$H-NMR (400 MHz, DMSO-D6): δ (ppm) = 2.31 (s, 3H), 2.61 (s, 6H), 5.97 (s, 0.33H), 7.24 (s, 2H), 7.46 (t, 1H), 7.84 (d, 1H), 7.91 (d, 1H), 8.24 (s, 1H).
$^{183}$W-NMR (20.84 MHz, DMSO-D6): δ (ppm) = -95.39 (s, 12W).
ESI-MS: POSITIVE m/z 400.9 ($[C_{15}H_{15}BrI]^{+}$)
NEGATIVE m/z 712.3 (median) ($[H_4W_{12}O_{40}]^{4-}$)

[Example 25: Hexakis((4-(octyloxy)phenyl)(phenyl)iodonium)metatungstate]

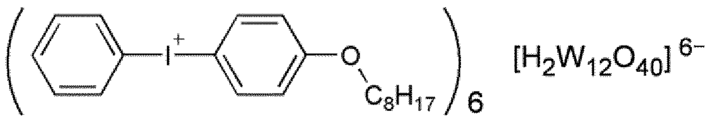

A target compound was produced in the same manner as in the salt exchange method in Example 1 except that (4-(octyloxy)phenyl)(phenyl)iodonium hexafluoroantimonate was used as a raw material compound instead of the bis(2-trifluoromethylphenyl)phenylsulfonium chloride.

$^{1}$H-NMR (400 MHz, DMSO-D6): δ (ppm) = 0.85 (t, 3H), 1.24-1.38 (m, 10H), 1.65-1.72 (m, 2H), 4.00 (t, 2H), 5.97 (s, 0.33H), 7.07 (d, 2H), 7.52 (t, 2H), 7.64 (t, 1H), 8.16-8.22 (m, 4H).
$^{183}$W-NMR (20.84 MHz, DMSO-D6): δ (ppm) = -95.39 (s, 12W).
ESI-MS: POSITIVE m/z 409.1 ($[C_{20}H_{26}IO]^{+}$)
NEGATIVE m/z 712.3 (median) ($[H_4W_{12}O_{40}]^{4-}$)

[Example 26: Hexakis((4-((2-hydroxytetradecyl)oxy)phenyl)(phenyl)iodonium)metatungstate]

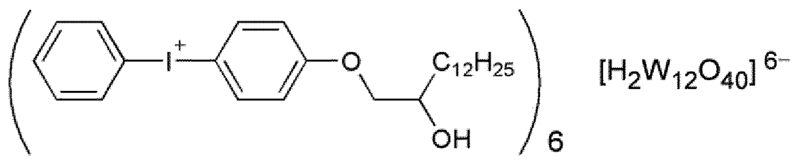

A target compound was produced in the same manner as in the salt exchange method in Example 1 except that (4-((2-hydroxytetradecyl)oxy)phenyl)(phenyl)iodonium hexafluoroantimonate was used as a raw material compound instead of the bis(2-trifluoromethylphenyl)phenylsulfonium chloride.

$^{1}$H-NMR (400 MHz, DMSO-D6): δ (ppm) = 0.85 (t, 3H), 1.23-1.47 (m, 22H), 3.69-3.76 (m, 1H), 3.83-3.91 (m, 2H), 4.84 (d, 1H), 5.97 (s, 0.33H), 7.07 (d, 2H), 7.52 (t, 2H), 7.62-7.66 (m, 1H), 8.16-8.22 (m, 4H).
$^{183}$W-NMR (20.84 MHz, DMSO-D6): δ (ppm) = -95.39 (s, 12W).
ESI-MS: POSITIVE m/z 509.2 ($[C_{26}H_{38}IO_2]^{+}$)
NEGATIVE m/z 712.3 (median) ($[H_4W_{12}O_{40}]^{4-}$)

[Example 27: Hexakis(mesityl(3-(trifluoromethyl)phenyl)iodonium)metatungstate]

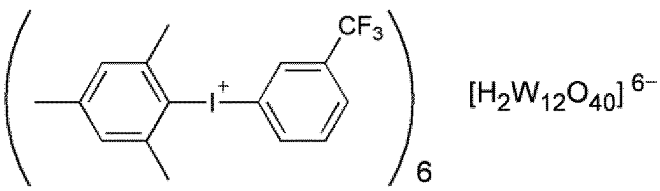

A target compound was produced in the same manner as in the salt exchange method in Example 1 except that mesityl(3-(trifluoromethyl)phenyl)iodonium triflate was used as a raw material compound instead of the bis(2-trifluoromethylphenyl)phenylsulfonium chloride.

$^1$H-NMR (400 MHz, DMSO-D6): δ (ppm) = 2.31 (s, 3H), 2.61 (s, 6H), 5.97 (s, 0.33H), 7.25 (s, 2H), 7.73 (t, 1H), 8.01 (d, 1H), 8.15 (d, 1H), 8.43 (s, 1H).
$^{183}$W-NMR (20.84 MHz, DMSO-D6): δ (ppm) = -95.39 (s, 12W).
ESI-MS: POSITIVE m/z 391.0 ($[C_{16}H_{15}F_3I]^+$)
NEGATIVE m/z 712.3 (median) ($[H_4W_{12}O_{40}]^{4-}$)

[Production Example 1: Bis(tetrabutylammonium)hexatungstate]

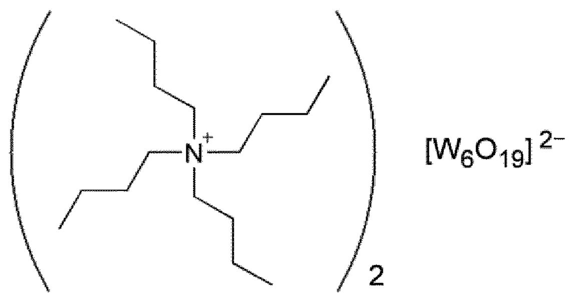

33.00 g of sodium tungstate·dihydrate, 40.00 g of acetic anhydride, and 30.00 g of dimethylformamide were added to a 300-cc flask and were stirred at 100°C for 3 hours. After completion of the reaction and air cooling, 20.00 g of acetic anhydride, 50.00 g of dimethylformamide, and 19.00 g of 12 N hydrochloric acid were added thereto and were stirred for approximately 30 minutes. The resulting reaction solution was filtered under reduced pressure to remove the filtrate, and a solution of 15.10 g of tetrabutylammonium bromide dissolved in 50.00 g of methanol was added to the filtrate and was stirred. The resulting solution was filtered under reduced pressure to collect a powder, thereby producing tetrabutylammonium hexatungstate ($((n\text{-}Bu)_4N^+)_2 \cdot [W_6O_{19}]^{2-}$) as a raw material compound.

[Example 28: Bis(bis(2-trifluoromethylphenyl)phenylsulfonium)hexatungstate]

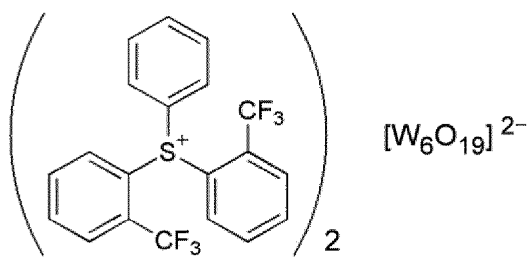

A target compound was produced in the same manner as in the salt exchange method in Example 1 except that tetrabutylammonium hexatungstate was used instead of the ammonium metatungstate as a raw material compound.

$^1$H-NMR (400 MHz, DMSO-D6): δ (ppm) = 7.65-8.35 (m, 13H).
$^{183}$W-NMR (20.84 MHz, DMSO-D6): δ (ppm) = +59.84 (s, 6W).
ESI-MS: POSITIVE m/z 399.1 ($[C_{20}H_{13}F_6S]^+$)
NEGATIVE m/z 703.8 (median) ($[W_6O_{19}]^{2-}$)

## Claims

**1.** An isopolyoxotungstate compound represented by the general formula (I) or a solvate thereof:

$(A^{m+})_{x/m}(B^{n+})_{y/n}(C^{-(x+y)})$ (I)

wherein

$A^{m+}$ each independently denotes $H^+$, a metal ion, or an ammonium ion;
$B^{n+}$ each independently denotes a sulfonium cation or an iodonium cation;
$C^{-(x+y)}$ denotes an isopolyoxotungstic acid; and
m denotes an integer in the range of 1 to 5, n denotes an integer of 1 or 2, x and x/m denote an integer, and y and y/n denote a natural number.

2. The isopolyoxotungstate compound or a solvate thereof according to claim 1, wherein the isopolyoxotungstic acid is $[W_4O_{13}]^{2-}$, $[W_5O_{16}]^{2-}$, $[W_6O_{19}]^{2-}$, $[W_7O_{22}]^{2-}$, $[W_7O_{24}]^{6-}$, $[H_zW_{12}O_{40}]^{-(8-z)}$ (wherein z denotes an integer in the range of 1 to 4), $[W_{10}O_{32}]^{4-}$, $[H_4W_{11}O_{38}]^{6-}$, $[H_7W_{11}O_{40}]^{7-}$, $[HW_5O_{19}]^{7-}$, $[H_3W_{11}O_{22}]^{5-}$, $[H_4W_{22}O_{74}]^{12-}$, or $[H_{10}W_{34}O_{116}]^{18-}$.

3. An isopolyoxotungstate mixture comprising two or more different isopolyoxotungstate compounds or solvates thereof according to claim 1, wherein the $B^{n+}$ denotes a sulfonium cation.

4. The isopolyoxotungstate mixture according to claim 3, wherein a mole ratio of S to W in XRF analysis or XPS analysis is S:W = 1 to 8:4 to 12.

5. An isopolyoxotungstate mixture comprising two or more different isopolyoxotungstate compounds or solvates thereof according to claim 1, wherein the $B^{n+}$ denotes an iodonium cation.

6. The isopolyoxotungstate mixture according to claim 5, wherein a mole ratio of I to W in XRF analysis or XPS analysis is I:W = 1 to 8:4 to 12.

7. The isopolyoxotungstate compound or a solvate thereof according to claim 1, wherein the sulfonium cation is a sulfonium cation represented by the general formula (II-1):

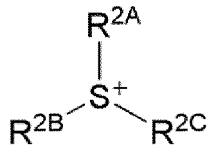

(I I − 1)

wherein

$R^{2A}$, $R^{2B}$, and $R^{2C}$ each independently denote an optionally substituted $C_{1-18}$ hydrocarbyl group, 3- to 18-membered non-aromatic heterocyclic group, or 5- to 18-membered aromatic heterocyclic group;
a divalent carbon atom at any position excluding a terminal of $R^{2A}$ to $R^{2C}$ may be replaced by -O-, -C(=O)-, COO-, -OCO-, -CONH-, -NHCO-, -S-, or $SO_2$- (provided that adjacent divalent carbon atoms are not replaced at the same time); and
any two of $R^{2A}$, $R^{2B}$, and $R^{2C}$ may be linked to each other directly by a single bond or via a divalent linking group -O-, -S-, -C(=O)-, -S(=O)-, $-S(=O)_2-$, -C(=O)O-, or a $C_{1-3}$ alkylene group to form a ring together with a sulfur atom in the formula (II-1), or
a sulfonium dication represented by the general formula (II-2):

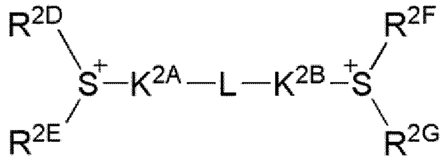

(I I − 2)

wherein
$R^{2D}$, $R^{2E}$, $R^{2F}$, and $R^{2G}$ each independently denote an optionally substituted $C_{1-18}$ hydrocarbyl group, 3- to 18-membered non-aromatic heterocyclic group, or 5- to 18-membered aromatic heterocyclic group;

a divalent carbon atom at any position excluding a terminal of $R^{2D}$, $R^{2E}$, $R^{2F}$, and $R^{2G}$ may be replaced by -O-, -C(=O)-, COO-, -OCO-, -CONH-, -NHCO-, -S-, or $SO_2$-(provided that adjacent divalent carbon atoms are not replaced at the same time);

$K^{2A}$, $K^{2B}$, and L each independently denote an optionally substituted $C_{1-18}$ hydrocarbylene group;

a divalent carbon atom at any position of $K^{2A}$, $K^{2B}$, and L may be replaced by -O-, - C(=O)-, COO-, -OCO-, -CONH-, -NHCO-, -S-, or $SO_2$- (provided that adjacent divalent carbon atoms are not replaced at the same time); and

any two of $R^{2D}$, $R^{2E}$, and $K^{2A}$ and/or any two of $R^{2F}$, $R^{2G}$, and $K^{2B}$ may be linked to each other directly by a single bond or via a divalent linking group -O-, -S-, -C(=O)-, - S(=O)-, -S(=O)$_2$-, -C(=O)O-, or a $C_{1-3}$ alkylene group to form a ring together with a sulfur atom in the formula (II-2).

**8.** The isopolyoxotungstate compound or a solvate thereof according to claim 1, wherein the sulfonium cation is a sulfonium cation represented by the general formula (III-1), (III-2), (III-3), (III-4), or (III-5),

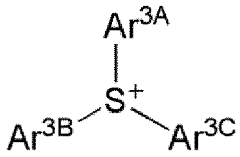

(III-1)

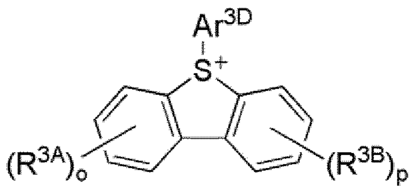

(III-2)

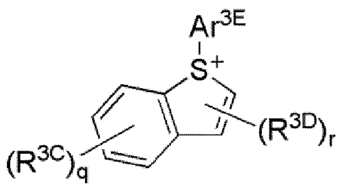

(III-3)

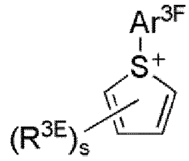

(III-4)

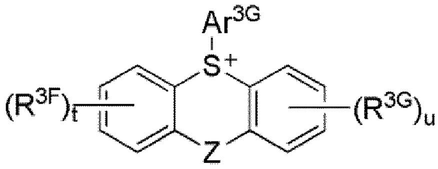

(III-5)

in the formulae (III-1) to (III-5),

$Ar^{3A}$, Ar3B, $Ar^{3C}$, $Ar^{3D}$, $Ar^{3E}$, $Ar^{3F}$, and $Ar^{3G}$ each independently denote an optionally substituted $C_{6-18}$ aryl group or 5- to 18-membered heterocyclic group,

in the optionally substituted $C_{6-18}$ aryl group or 5- to 18-membered heterocyclic group, at least part of hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a

$C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of hydrogen atoms may be substituted by a halogen atom, a hydroxy group, a thiol group, a nitro group, a cyano group, a carboxy group, an amino group, or a sulfo group, a divalent carbon atom at any position excluding a terminal of the substituent may be replaced by -O-, -C(=O)-, COO-, -OCO-, -CONH-, -NHCO-, -S-, or $SO_2$- (provided that adjacent divalent carbon atoms are not replaced at the same time);

$R^{3A}$, $R^{3B}$, $R^{3C}$, $R^{3D}$, $R^{3E}$, $R^{3F}$, and $R^{3G}$ each independently denote a halogen atom, a hydroxy group, a thiol group, a nitro group, a cyano group, a carboxy group, an amino group, or a sulfo group, or each independently denote a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of hydrogen atoms may be substituted by a halogen atom, a hydroxy group, a thiol group, a nitro group, a cyano group, a carboxy group, an amino group, or a sulfo group,

the divalent carbon atom at any position excluding the terminal may be replaced by -O-, -C(=O)-, COO-, -OCO-, -CONH-, -NHCO-, -S-, or $SO_2$- (provided that adjacent divalent carbon atoms are not replaced at the same time);

o, p, q, s, t, and u each denote an integer in the range of 0 to 4, r denotes an integer in the range of 0 to 2, and when o to u denote an integer of 2 or more, $R^{3D}$, $R^{3E}$, $R^{3F}$, $R^{3G}$, $R^{3H}$, $R^{3I}$, and $R^{3J}$ may be the same or different; and

Z denotes -O-, -S-, -C(=O)-, -S(=O)-, -S(=O)$_2$-, -C(=O)O-, or a $C_{1-3}$ alkylene group.

**9.** The isopolyoxotungstate compound or a solvate thereof according to claim 1, wherein the iodonium cation is an iodonium cation represented by the general formula (IV):

$$R^{4A}-\overset{+}{I}-R^{4B} \qquad (IV)$$

wherein

$R^{4A}$ and $R^{4B}$ each independently denote an optionally substituted $C_{1-18}$ hydrocarbyl group, an optionally substituted 3- to 18-membered non-aromatic heterocyclic group, or an optionally substituted 5- to 18-membered aromatic heterocyclic group, and

$R^{4A}$ and $R^{4B}$ may be linked to each other directly by a single bond or via a divalent linking group -O-, -S-, -C(=O)-, -S(=O)-, -S(=O)$_2$-, -C(=O)O-, or a $C_{1-3}$ alkylene group to form a ring together with an iodine atom in the formula (V-1).

**10.** The isopolyoxotungstate compound or a solvate thereof according to claim 1, wherein the iodonium cation is an iodonium cation cation represented by the general formula (V-1) or (V-2):

$$Ar^{5A}-\overset{+}{I}-Ar^{5B} \qquad (V-1)$$

wherein

$Ar^{5A}$ and $Ar^{5B}$ each independently denote an optionally substituted $C_{6-18}$ aryl group or 5- to 18-membered heterocyclic group,

in the optionally substituted $C_{6-18}$ aryl group or 5- to 18-membered heterocyclic group, at least part of hydrogen atoms may be substituted by a halogen atom, a hydroxy group, a thiol group, a nitro group, a cyano group, a carboxy group, an amino group, or a sulfo group, or the optionally substituted $C_{6-18}$ aryl group or 5- to 18-membered heterocyclic group may be substituted by a $C_{1-18}$ hydrocarbyl group, a $C_{1-18}$ hydrocarbyloxy group, a

$C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of hydrogen atoms may be substituted by a halogen atom, a hydroxy group, a thiol group, a nitro group, a cyano group, a carboxy group, an amino group, or a sulfo group, a divalent carbon atom at any position excluding a terminal of the substituent may be replaced by -O-, -C(=O)-, COO-, -OCO-, -CONH-, -NHCO-, -S-, or $SO_2$- (provided that adjacent divalent carbon atoms are not replaced at the same time); and

$Ar^{5A}$ and $Ar^{5B}$ may be linked to each other directly by a single bond or via a divalent linking group -O-, -S-, -C(=O)-, -S(=O)-, $-S(=O)_2-$, -C(=O)O-, or a $C_{1-3}$ alkylene group to form a ring together with an iodine atom in the formula (V-1);

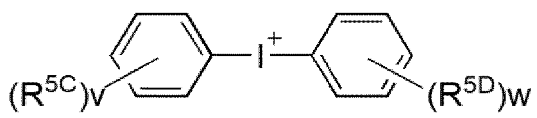
(V − 2)

wherein

$R^{5C}$ and $R^{5D}$ each independently denote a halogen atom, a hydroxy group, a thiol group, a nitro group, a cyano group, a carboxy group, an amino group, or a sulfo group, or each independently denote a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, a $C_{1-18}$ hydrocarbylamino group, a di-$C_{1-18}$ hydrocarbylamino group, a $C_{1-18}$ hydrocarbylaminocarbonyl group, a di-$C_{1-18}$ hydrocarbylaminocarbonyl group, a $C_{1-18}$ hydrocarbylcarbonylamino group, a $C_{1-18}$ hydrocarbylaminocarbonyloxy group, a di-$C_{1-18}$ hydrocarbylaminocarbonyloxy group, a $C_{1-18}$ hydrocarbylaminocarbonylamino group, a di-$C_{1-18}$ hydrocarbylaminocarbonylamino group, a $C_{1-18}$ hydrocarbyloxycarbonylamino group, a $C_{1-18}$ hydrocarbylthio group, a $C_{1-18}$ hydrocarbylsulfinyl group, or a $C_{1-18}$ hydrocarbylsulfonyl group, in which at least part of hydrogen atoms may be substituted by a halogen atom, a hydroxy group, a thiol group, a nitro group, a cyano group, a carboxy group, an amino group, or a sulfo group,

the divalent carbon atom at any position excluding the terminal may be replaced by -O-, -C(=O)-, COO-, -OCO-, -CONH-, -NHCO-, -S-, or $SO_2$- (provided that adjacent divalent carbon atoms are not replaced at the same time); and

v and w each denote an integer in the range of 0 to 5, and when v and w denote an integer of 2 or more, $R^{5C}$ and $R^{5D}$ may be the same or different.

**11.** A method for producing an isopolyoxotungstate compound represented by the general formula (I) or a solvate thereof, the method comprising:

reacting a compound represented by the general formula (VI-1) with a compound represented by the general formula (VI-2) to perform salt exchange;

$(A^{m+})_{x/m}(B^{n+})_{y/n}(C^{-(x+y)})$ (I)

$(A^{m+})_{x/m}(A'^{m'+})_{y/m'}(C^{-(x+y)})$ (VI-1)

$(B^{n+})_{y/n}(Y^{-})_{y}$ (VI-2)

wherein

$A^{m+}$ each independently denotes $H^+$, a metal ion, or an ammonium ion;

$A'^{m'+}$ each independently denotes a metal ion or an ammonium ion;

$B^{n+}$ each independently denotes a sulfonium cation or an iodonium cation;

$C^{-(x+y)}$ denotes an isopolyoxotungstic acid;

$Y^-$ denotes a monovalent counter anion; and

m and m' denote an integer in the range of 1 to 5, n denotes an integer of 1 or 2, x and x/m denote an integer, and y/m', y, and y/n denote a natural number.

12. A method for producing an isopolyoxotungstate compound represented by the general formula (I) or a solvate thereof, the method comprising:

reacting a compound represented by the general formula (VI-1) with a compound represented by the general formula (VI-2) to perform salt exchange; and
removing a compound represented by the general formula (VI-3) from the resulting compounds represented by the general formulae (I) and (VI-3);

$$(A^{m+})_{x/m}(B^{n+})_{y/n}(C^{-(x+y)}) \qquad (I)$$

$$(A^{m+})_{x/m}(A'^{m'+})_{y/m'}(C^{-(x+y)}) \qquad (VI\text{-}1)$$

$$(B^{n+})_{y/n}(Y^{-})_{y} \qquad (VI\text{-}2)$$

$$(A'^{m'+})_{y/m'}(Y^{-})_{y} \qquad (VI\text{-}3)$$

wherein
$A^{m+}$ each independently denotes $H^+$, a metal ion, or an ammonium ion;
$A'^{m'+}$ each independently denotes a metal ion or an ammonium ion;
$B^{n+}$ each independently denotes a sulfonium cation or an iodonium cation;
$C^{-(x+y)}$ denotes an isopolyoxotungstic acid;
$Y^-$ denotes a monovalent counter anion; and
m and m' denote an integer in the range of 1 to 5, n denotes an integer of 1 or 2, x and x/m denote an integer, and y/m', y, and y/n denote a natural number.

13. A method for producing an isopolyoxotungstate compound represented by the general formula (I) or a solvate thereof, the method comprising:

ion-exchanging $A'^{m'+}$ of a compound represented by the general formula (VI-1) with $H^+$ using an ion-exchange resin; and
reacting the resulting compound represented by the general formula (VI-4) with a compound represented by the general formula (VI-2);

$$(A^{m+})_{x/m}(B^{n+})_{y/n}(C^{-(x+y)}) \qquad (I)$$

$$(A^{m+})_{x/m}(A'^{m'+})_{y/m'}(C^{-(x+y)}) \qquad (VI\text{-}1)$$

$$(A^{m+})_{x/m}(H^{+})_{y}(C^{-(x+y)} \qquad (VI\text{-}4)$$

$$(B^{n+})_{y/n}(Y^{-})_{y} \qquad (VI\text{-}2)$$

wherein
$A^{m+}$ each independently denotes $H^+$, a metal ion, or an ammonium ion;
$A'^{m'+}$ each independently denotes a metal ion or an ammonium ion;
$B^{n+}$ each independently denotes a sulfonium cation or an iodonium cation;
$C^{-(x+y)}$ denotes an isopolyoxotungstic acid;
$Y^-$ denotes a monovalent counter anion; and

m and m' denote an integer in the range of 1 to 5, n denotes an integer of 1 or 2, x and x/m denote an integer, and y/m', y, and y/n denote a natural number.

## INTERNATIONAL SEARCH REPORT

International application No.
**PCT/JP2023/042798**

**A. CLASSIFICATION OF SUBJECT MATTER**

***C07F 11/00***(2006.01)i; ***C07C 25/02***(2006.01)i; ***C07C 381/12***(2006.01)i
FI: C07F11/00 C; C07C25/02; C07C381/12 CSP

According to International Patent Classification (IPC) or to both national classification and IPC

**B. FIELDS SEARCHED**

Minimum documentation searched (classification system followed by classification symbols)

C07F11/00; C07C25/02; C07C381/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | MOKBEL, H. et al., Iodonium-polyoxometalate and thianthrenium-polyoxometalate as new one-component UV photoinitiators for radical and cationic polymerization, Journal of Polymer Science Part A: Polymer Chemistry, 01 February 2015, vol. 53, no. 8, pp. 981-989, doi:10.1002/pola.27526<br>Abstract, p. 982, left column, line 3 from the bottom to p. 982, right column, line 17, p. 983, right column, lines 6-40, p. 983, right column, lines 3-10, p. 988, left column, line 12 from the bottom to p. 988, right column, line 5, Schemes 1, 2 | 1-12 |
| Y | | 1-13 |
| Y | LIU, Y.-J. et al., Recent advances in isopolyoxotungstates and their derivatives, Acta Crystallographica Section C: Structural Chemistry, 18 October 2018, vol. 74, no. 11, pp. 1202-1221, doi:10.1107/S 2053229618012524<br>Table 1, fig. 3-5 | 1-12 |

☑ Further documents are listed in the continuation of Box C. ☐ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 February 2024** | **20 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No. **PCT/JP2023/042798**

**C. DOCUMENTS CONSIDERED TO BE RELEVANT**

| Category* | Citation of document, with indication, where appropriate, of the relevant passages | Relevant to claim No. |
|---|---|---|
| Y | KAR, A. et al., Post-functionalization through covalent modification of organic counter ions: a stepwise and controlled approach for novel hybrid polyoxometalate materials, Dalton Transactions, 28 August 2020, vol. 49, pp. 12174-12179, doi:10.1039/d 0dt 01410a<br>p. 12174, right column, line 8 from the bottom to p. 12175, right column, line 4, p. 12176, right column, lines 8-18, fig. 1, 2, hybrids 1, 3 | 1-12 |
| Y | KUMAR, A. et al., Engineering Multifunctionality in Hybrid Polyoxometalates: Aromatic Sulfonium Octamolybdates as Excellent Photochromic Materials and Self-Separating Catalysts for Epoxidation, Inorganic Chemistry, 17 August 2017, vol. 56, no. 17, pp. 10325-10336, doi:10.1021/acs.inorgchem.7b 01143<br>abstract, p. 10326, right column, line 12 to p. 10326, left column, line 11, p. 10333, right column, lines 43-54, Chart 1, fig. 2 | 1-12 |
| Y | HAKOUK, K. et al., Sulfonium polyoxometalates: a new class of solid-state photochromic hybrid organic-inorganic materials, Inorganic Chemistry, 08 January 2013, vol. 52, no. 2, pp. 555-557, doi:10.1021/ic 302477p<br>Abstract, p. 555, right column, line 4 to p. 556, left column, line 15, disclosure of "Synthesis of (Me3S)4[Mo8O26](2)." in "Supporting Information" | 1-12 |
| Y | NOZAKI, KATO C. et al., Synthesis, X-ray Crystal Structure, and Photochromism of a Sandwich-Type Mono-Aluminum Complex Composed of Two Tri-Lacunary α-Dawson-Type Polyoxotungstates, Materials, 26 July 2019, vol. 12, no. 15, Articles No. 2383, pp. 1-13, doi:10.3390/ma12152383<br>abstract, p. 2, line 10 from the bottom to p. 3, line 15, p. 3, lines 11-5 from the bottom | 13 |

Form PCT/ISA/210 (second sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 11192908 B


### Non-patent literature cited in the description


- **YA-JIE LIU et al.** Recent advances in isopolyoxotungstates and their derivatives. *Acta Crystallographica*, 2018, vol. C74, 1202-1221